(19) Europäisches Patentamt
European Patent Office
Office européen des brevets

(11) Publication number : **0 487 270 A2**

(12) **EUROPEAN PATENT APPLICATION**

(21) Application number : **91310590.4**

(22) Date of filing : **15.11.91**

(51) Int. Cl.[5] : **C07C 271/22,** A61K 31/27, C07F 9/40, A61K 31/66, C07C 323/60

(30) Priority : **19.11.90 US 615683**
**16.08.91 US 747156**

(43) Date of publication of application :
**27.05.92 Bulletin 92/22**

(84) Designated Contracting States :
**AT BE CH DE DK ES FR GB IT LI LU NL SE**

(71) Applicant : **MERCK & CO. INC.**
**126, East Lincoln Avenue P.O. Box 2000**
**Rahway New Jersey 07065-0900 (US)**

(72) Inventor : **Graham, Samuel L.**
**325 Widlund Drive**
**Schwenksville, PA 19473 (US)**
Inventor : **Hungate, Randall W.**
**1925 Rampart Lane**
**Lansdale, PA 19446 (US)**
Inventor : **Huff, Joel R.**
**825 Surrey Drive**
**Gwynedd Valley, PA 19437 (US)**
Inventor : **Lyle, Terry A.**
**570 Camp WaWa Road**
**Lederach, PA 19450 (US)**
Inventor : **Young, Steven D.**
**1280 Mark Drive**
**Lansdale, PA 19446 (US)**
Inventor : **Britcher, Susan F.**
**735 Port Indian Road**
**Norristown, PA 19403 (US)**
Inventor : **Scholz, Thomas H.**
**127 South Front Street**
**Souderton, PA 18964 (US)**
Inventor : **Payne, Linda S.**
**1502 Henning Way**
**Landsdale, PA 19446 (US)**
Inventor : **Dorsey, Bruce D.**
**203 Continental Drive**
**Harleysville, PA 19438 (US)**

(74) Representative : **Thompson, John Dr. et al**
**Merck & Co., Inc. European Patent**
**Department Terlings Park Eastwick Road**
**Harlow, Essex CM20 2QR (GB)**

(54) **HIV protease inhibitors having polyether substituents.**

(57) Polyether derivatives of the form,

A-G-B-B-J

wherein G is a dipeptide isostere substituted with a polyether, B an amino acid or analog thereof, and J a small terminal group are described. These compounds are useful in the inhibition of DIV protease, the prevention or treatment of infection by HIV and the treatment of AIDS, either as compounds, pharmaceutically acceptable salts, pharmaceutical composition ingredients, whether or not in combination with other antivirals, immunomodulators, antibiotics or vaccines. Methods of treating AIDS and methods of preventing or treating infection by HIV are also described.

EP 0 487 270 A2

The present invention is concerned with compounds which inhibit the protease encoded by human immunodeficiency virus (HIV). The compounds, or pharmaceutically acceptable salts thereof, are of value in the prevention of infection by HIV, the treatment of infection by HIV and the treatment of the resulting acquired immune deficiency syndrome (AIDS).

It also relates to pharmaceutical compositions containing the compounds and to a method of use of the present compounds and other agents for the treatment of AIDS & viral infection by HIV.

## BACKGROUND OF THE INVENTION

A retrovirus designated human immunodeficiency virus (HIV) is the etiological agent of the complex disease that includes progressive destruction of the immune system (acquired immune deficiency syndrome; AIDS) and degeneration of the central and peripheral nervous system. This virus was previously known as LAV, HTLV-III, or ARV. A common feature of retrovirus replication is the extensive post-translational processing of precursor polyproteins by a virally encoded protease to generate mature viral proteins required for virus assembly and function. Interruption of this processing appears to prevent the production of normally infectious virus. For example, Crawford, S. et al., J. Virol., 53, 899, 1985, demonstrated that genetic deletion mutations of the protease in murine leukemia virus which prevent processing of precursor structural proteins result in non-infectious viral particles. Unprocessed structural proteins also have been observed in clones of non-infectious HIV strains isolated from human patients. These results suggest that inhibition of the HIV protease represents a viable method for the treatment of AIDS and the prevention or treatment of infection by HIV.

Nucleotide sequencing of HIV shows the presence of a pol gene in one open reading frame [Ratner, L. et al., Nature, 313, 277(1985)]. Amino acid sequence homology provides evidence that the pol sequence encodes reverse transcriptase, an endonuclease and an HIV protease [Toh, H. et al., EMBO J. 4, 1267 (1985); Power, M.D. et al., Science, 231, 1567 (1986); Pearl, L.H. et al., Nature 329, 351 (1987)]. Applicants demonstrate that the compounds of this invention are inhibitors of HIV protease.

The particular advantages of the compounds of the present invention are their potency and bioavailability. The polyether substituents increase solubility, another advantage of the present invention.

## BRIEF DESCRIPTION OF THE INVENTION

Compounds of formula I, as herein defined, are disclosed. These compounds are useful in the inhibition of HIV protease, the prevention of infection by HIV, the treatment of infection by HIV and in the treatment of AIDS, either as compounds, pharmaceutically acceptable salts, pharmaceutical composition ingredients, whether or not in combination with other antivirals, immunomodulators, antibiotics or vaccines. Methods of treating AIDS, methods of preventing infection by HIV, and methods of treating infection by HIV are also disclosed.

## ABBREVIATIONS

| Designation | Activating Agent |
|---|---|
| HBT (HOBT or HOBt) | 1-hydroxybenzotriazole hydrate |
| | Condensing Agent |
| EDC | 1-ethyl-3-(3-dimethylamino-propyl)carbodiimide |
| | Deprotonating Agent |
| LDA | lithium diisopropylamide |

| | Other Reagents |
|---|---|
| $Bu_4N^+F^-$ | tetrabutylammonium fluoride |
| DME | dimethoxyethane |
| DMF | dimethylformamide |
| MCPBA | m-chloroperoxybenzoic acid |
| TBAF | tetrabutylammonium fluoride |
| TBDMSCl | t-butyldimethylsilyl chloride |

## DETAILED DESCRIPTION OF THE INVENTION AND PREFERRED EMBODIMENTS

This invention is concerned with the use of compounds of formula I, combinations thereof, or pharmaceutically acceptable salts thereof, in the inhibition of HIV protease, the prevention or treatment of infection by HIV and in the treatment of the resulting acquired immune deficiency syndrome (AIDS). Compounds of formula I are defined as follows:

A-G-B-B-J I,

wherein A is:

1) hydrogen;

2) $R^1-\overset{O}{\overset{\|}{C}}-$

wherein $R^1$ is

a) $C_{1-6}$ alkyl either unsubstituted or substituted with one or more of

i) $C_{1-4}$ alkyl;

ii) hydroxy;

iii) carboxy;

iv) halo wherein halo is F, C1, Br, or I:

v) amino;

vi) $C_{1-3}$ alkoxycarbonyl;

vii) $C_{1-3}$ alkoxy;

viii) $-CONR^2R^3$ wherein $R^2$ and $R^3$ are the same or different and are hydrogen, $C_{1-5}$ alkyl or $C_{1-5}$ alkoxyalkyl or joined together either directly to form a 5-7 membered heterocycle, or through a heteroatom selected from N, O, and S, to form a 6-membered heterocycle with the nitrogen to which they are attached;

ix) $-NR^2R^3$;

x) $-\underset{R}{\underset{|}{N}}-D-R^4$ wherein,

R is hydrogen or $C_{1-4}$ alkyl,

D is $-\overset{O}{\overset{\|}{C}}-$, $-\overset{S}{\overset{\|}{C}}-$, $-\underset{O}{\underset{\|}{\overset{O}{\overset{\|}{S}}}}-$, $-\overset{NR}{\overset{\|}{C}}-$, and

$R^4$ is NHR, $C_{1-3}$ alkyl, $C_{1-4}$ alkoxy, or $NR^2R^3$;

xi) $C_{3-7}$ cycloalkyl or $C_{6-10}$ aryl;

xii) 5 or 6 membered heterocycle, unsubstituted or substituted with -OH, NHR, or $C_{1-4}$ alkyl; or

xiii) aryl of 6-10 carbon atoms, either unsubstituted or substituted with one or more of

(a) halo,

(b) hydroxy,

(c) $C_{1-3}$ alkoxy,

(d) $C_{1-3}$ alkyl,

(e) $-NR_2$, wherein R is defined above,

(f) 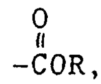

(g) 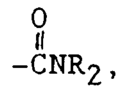

(h) $-SO_2NR_2$,

(i) $-CH_2NR_2$,

(j) 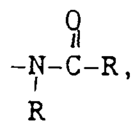 or

(k) 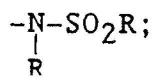

xiv) $X[(CH_2)_mO]_nR$ where $X=CH_2$, NR, O, S, SO or $SO_2$; n=1-6, m may be 1-3 within each repeating unit n, and R is defined above;

b) aryl of 6-10 carbon atoms, either unsubstituted or substituted with one or more of

i) $C_{1-4}$ alkyl,

ii) $C_{1-3}$ alkoxy,

iii) hydroxy, or

iv) halo;

v) $-NR_2$,

vi) 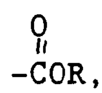

vii) 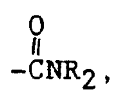

viii) $-SO_2NR_2$,

ix) $-CH_2NR_2$,

x) -NRCOR, or

xi) $-NRSO_2R$;

xii) $X[(CH_2)_mO]_nR$ where $X=CH_2$, NR, O, S, SO or $SO_2$; n=1-6, m may be 1-3 within each repeating unit n, and R is defined above;

xiii) $CH_2X[(CH_2)_mO]_nR$;

c) 5 or 6 membered heterocycle;

3) $R^1-SO_2-$,

4) 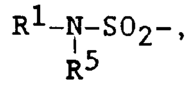

wherein

$R^5$ is H or $C_{1-5}$ alkyl or joined together with $R^1$ either directly to form 5-7 membered heterocycle, or through a heteroatom selected from N, O, and S, to form a 6-membered heterocycle with the nitrogen to which they are attached;

5) 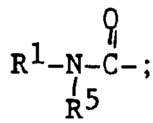

6) 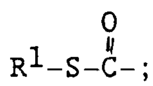

7) 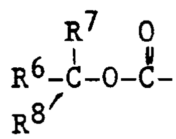

wherein $R^6$, $R^7$, and $R^8$ are independently

a) H,

b) $C_{1-6}$ alkyl unsubstituted or substituted with one or more of

i) halo,

ii) OH,

iii) aryl $SO_2$-,

iv) $-O-[(CH_2)_mO]_n-R$,

c) Aryl unsubstituted or substituted with one or more of

i) $C_{1-4}$ alkyl,

ii) $C_{1-3}$ alkoxy,

iii) halo,

iv) nitro,

v) acetoxy,

vi) dimethylaminocarbonyl,

vii) phenyl,

viii) $C_{1-3}$ alkoxycarbonyl

d) fluorenyl,

e) $R^6$, $R^7$, and $R^8$ may be independently joined to form a monocyclic, bicyclic, or tricyclic ring system which is $C_{3-10}$ cycloalkyl and may be substituted with $C_{1-4}$ alkyl, or

f) a 5-7 membered heterocycle;

G is

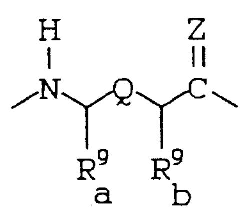

wherein Z is O, S, or NH and
$R^9_a$ or $R^9_b$ is independently

1) 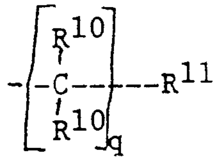, or

2) $C_{1-4}$alkylene -$R^{11}$;

wherein q is 0-5 and $R^{10}$ is independently

a) hydrogen,
b) hydroxy, or
c) $C_{1-4}$alkyl;

provided that $R^9_a$ or $R^9_b$ is always substituted with $R^{12}$;
$R^{11}$ is

a) $C_6$-$C_{10}$ aryl, unsubsituted or substituted with $R^{12}$ and optionally with one or more of
i) halo,
ii) $C_{1-4}$alkyl,
iii) $C_{1-3}$ alkoxy;

b) monocyclic or bicyclic aromatic heterocyle containing from 1 to 3 heteroatoms chosen from N,O,S and which may be substituted with $R^{12}$ and optionally with one or more of
i) halo,
ii) $C_{1-4}$ alkyl,
iii) $C_{1-3}$ alkoxy;

c) $C_{1-6}$ alkyl or $C_{1-6}$ alkenyl unsubstituted or substituted with $R^{12}$ and optionally with one or more of
i) hydroxy,
ii) $C_{1-4}$ alkyl,
iii) $-NH_2$, $-NHR$, $-NR_2$,

iv) 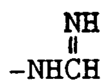,

v) 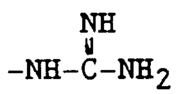,

vi) -COOH,

vii) 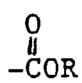,

viii) SR, S(O)R and $S(O_2)R$,
ix) $-SO_2NHR$,
x) $C_{1-4}$ alkyl sulfonyl amino or aryl sulfonyl amino,
xi) -CONHR,

xii) 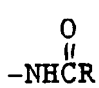,

xiii) -OR,
xiv) aryl $C_{1-3}$ alkoxy or,
xv) aryl;

d) $C_{3-7}$ cycloalkyl unsubstituted or substituted with $R^{12}$, and optionally with one or more of

i) hydroxy,
ii) $C_{1-4}$ alkyl,
iii) $-NH_2$, -NHR, $-NR_2$,

iv) 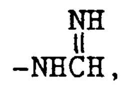

v) 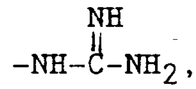

vi) -COOH,

vii) 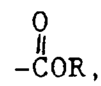

viii) SR, S(O)R and $S(O_2)R$,
ix) $-SO_2NHR$,
x) $C_{1-4}$ alkyl sulfonyl amino or aryl sulfonyl amino,
xi) -CONHR,

xii) 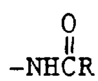 and

$R^{12}$ is
a) $X[((CH_2)_mO)_n]R^{13}$ where $X=CH_2$, NR, O, S, SO or $SO_2$; n=1-6, m may be 1-3 within each repeating unit n, and $R^{13}$ is selected from
i) H or $C_{1-4}$ alkyl,
ii) $C(=O)R^1$,
iii) $C(=O)X'[(CH_2)_mO]_nR$ wherein X′ is NR or O,
iv) $P(=O)(OM)_2$ where M is a mono or divalent metal ion,
v) $C(=O)OR^1$,
vi) $R^1R^5NC(=O)$;
b) $CH_2X[((CH_2)_mO)_n]R^{13}$,
$R^{14}$ is -OH or $-NHR^{15}$, wherein $R^{15}$ is -H,

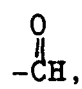

$-C_{1-4}$- alkyl, or -COOR; and
Q is

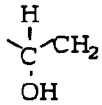 ; 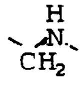 ; 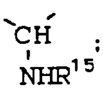 ; 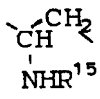 ; 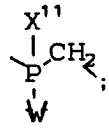 ; 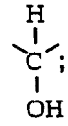 ; or 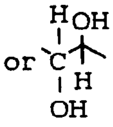 ;

wherein $R^{15}$ is defined above;
$X^{11}$ is O, S or NH; and
W is

1) OH,
2) $NH_2$,
3) OR, or
4) NHR;

B is, independently, absent, or

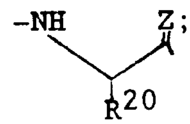

where $R^{20}$ is a hydrophobic group and is
a) $-CH(CH_3)_2$
b) $-CH(CH_3)(CH_2CH_3)$, or
c) -Phenyl;

J is
1) $YR^{16}$ wherein:
Y is O or NH, and
$R^{16}$ is
a) H;
b) $C_{1-6}$ alkyl, unsubstituted or substituted with one or more of
i) $-NR_2$,
ii) -OR,
iii) $-NHSO_2C_{1-4}$ alkyl,
iv) $-NHSO_2$ aryl, or $-NHSO_2$(dialkylaminoaryl),
v) $-CH_2OR$,
vi) $-C_{1-4}$ alkyl,

vii) 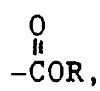

viii) 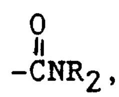

ix) 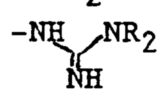 or 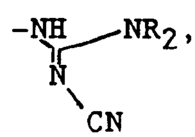

x) 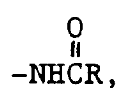

xi) 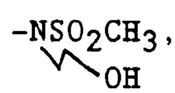

xii) 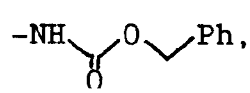

xiii) $-NR_3\oplus A\ominus$ wherein $A\ominus$ is a counterion,
xiv) $-NR^{17}R^{18}$ wherein $R^{17}$ and $R^{18}$ are the same or different and are $C_{1-5}$ alkyl joined together directly to form a 5-7 membered heterocycle containing up to one additional heteroatom selected from -O-, -S-, or -NR-,
xv) aryl,
xvi) -CHO,
xvii) $-OP(O)(OR_x)_2$ wherein $R_x$ is H or aryl, or

xvii) 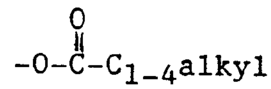

substituted with one or more of amine or quaternary amine, or -O-[$(CH_2)_mO]_n$-R, or -OP(O)$(OR_x)_2$;

c) -[$(CH_2)_mO]_nCH_3$ or -[$(CH_2)_mO]_n$ H;

2) $N(R^{16})_2$;

3) $-NR^{17}R^{18}$ wherein $R^{17}$ and $R^{18}$ are defined

4) 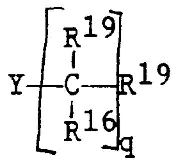

wherein:

Y, $R^{16}$ and q are defined above, and

$R^{19}$ is

a) hydrogen;

b) aryl unsubstituted or substituted with one or more of

i) halo,

ii) -OR, wherein R is H or $C_{1-4}$ alkyl,

iii) 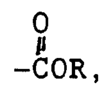

iv) 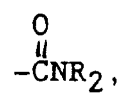

v) $-CH_2NR_2$,

vi) $-SO_2NR_2$,

vii) $-NR_2$,

viii) 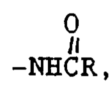

xi) $C_{1-4}$ alkyl,

x) phenyl

xi) $-CF_3$,

xii) 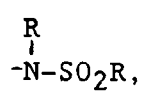

xiii) $-C_{1-4}$ alkyl $-NR_2$,

xiv) -OP(O)$(OR_x)_2$ wherein $R_x$ is H or aryl, or

xv) 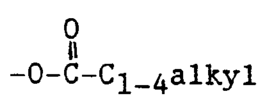

substituted with one or more of amine or quaternary amine, or $-OP(O)(OR_x)_2$;

c) A 5-7 membered heterocyclic ring or, 7-10 membered bicyclic heterocyclic ring which is saturated or unsaturated, the ring being unsubstituted or substituted with one or more of

i) halo,

ii) -OR, wherein R is H, $C_{1-4}$alkyl, or $C_{1-4}$alkenyl,

iii) $-\overset{O}{\overset{\|}{C}}OR$,

iv) $-\overset{O}{\overset{\|}{C}}NR_2$,

v) $-CH_2NR_2$,

vi) $-SO_2NR_2$,

vii) $-NR_2$,

viii) $-NH\overset{O}{\overset{\|}{C}}R$,

xi) $C_{1-4}$ alkyl,

x) phenyl

xi) $-CF_3$,

xii) $-\overset{R}{\overset{|}{N}}-SO_2R$,

xiii) phenyl $C_{1-4}$ alkyl,

xiv) $-OP(O)(OR_x)_2$ wherein $R_x$ is H or aryl,

xv) $-O-\overset{O}{\overset{\|}{C}}-C_{1-4}alkyl$

substituted with one or more of amine or quaternary amine, or $-OP(O)(OR_x)_2$, or $-O[(CH_2)_mO]_n-R$,

xvi) $-O-\overset{O}{\overset{\|}{C}}-O-[(CH_2)_mO]_n-R$, or

xvii) When sulfur or nitrogen are incorporated into the heterocycle, oxidized forms of S or N, i.e., sulfoxides, benzothiopyranylsulfone, sulfones or N-oxides are also included.

d) A 5 to 7 membered carbocyclic or 7-10 membered bicyclic carbocyclic ring which is either saturated or unsaturated, the carbocyclic ring being unsubstituted or substituted with one or more of

i) halo,

ii) -OR, wherein R is H or $C_{1-4}$ alkyl,

iii) $-\overset{O}{\overset{\|}{C}}OR$,

iv) $-\underset{O}{\underset{\|}{C}}NR_2$,

v) $-CH_2NR_2$,
vi) $-SO_2NR_2$,
vii) $-NR_2$,

viii) 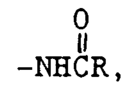

xi) $C_{1-4}$ alkyl,
x) phenyl,
xi) $-CF_3$,

xii) 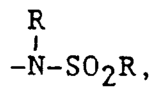

xiii) $-OP(O)(OR_x)_2$ wherein $R_x$ is H or aryl,

xiv) 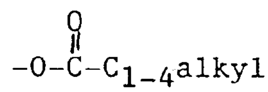

substituted with one or more of amine or quaternary amine, $-OP(O)(OR_x)_2$, or $-O-[(CH_2)_mO]_n-R$, or

xv) 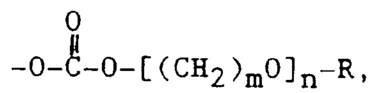

or pharmaceutically acceptable salts thereof.

In one embodiment of this invention,

A is

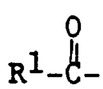 or 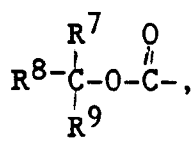 and

G is

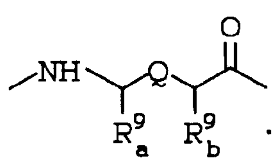

A second embodiment is further limited, wherein B is absent or present once and

Q is

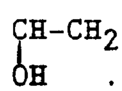

A third embodiment is further limited, wherein B is absent, J is $NHR^{19}$ and $R^{19}$ is a substituted 7- to 10-membered bicyclic carbocycle or heterocycle which may be saturated or unsaturated.

A fourth embodiment is further limited to J as indanyl, benzo[c]thiopyranyl-2,2-dioxide or cyclopentyl, any of which is unsubstituted or substituted.

Novel compounds of the invention include, but are not limited to:

N-(2(R)-hydroxy-1(S)-indanyl)-5(S)-(1,1-dimethylethyloxycarbonylamino)-4(S)-hydroxy-2(R)-(4-(1,4,7-trioxaoctyl)phenyl)methyl-6-phenylhexanamide,

N-(2(R)-hydroxy-1(S)-indanyl)-5(S)-(1,1-dimethylethoxycarbonyl)amino-4(S)-hydroxy-6-phenyl-2(R)-4-(2-hydroxyethoxy)phenylmethylhexanamide,

N-(2(R)-hydroxy-1(S)-indanyl)-5(S)-(1,1-dimethylethoxycarbonyl)amino-4(S)-hydroxy-6-phenyl-2(R)-4-(2-hydroxyethyl)phenylmethylhexanamide,

N-(2(R)-hydroxy-1(S)-indanyl)-5(S)-(1,1-dimethylethoxycarbonyl)amino-4(S)-hydroxy-6-phenyl-2(R)-4-(3-hydroxypropyl)phenylmethylhexanamide,

N-(2(R)-hydroxy-1(S)-indanyl)-5(S)-(1,1-dimethylethoxycarbonyl)amino-4(S)-hydroxy-6-phenyl-2(R)-4-(3-(2-methoxyethoxy)methoxypropyl)phenylmethylhexanamide,

[5(S)-(1,1-dimethylethoxycarbonylamino)-4(S)-hydroxy-6-phenyl-2(R)-4-(2-hydroxyethoxy)phenylmethylhexanoyl]-valine-N-3,6,9,12-tetraoxatridecylamide,

[5(S)-(1,1-dimethylethoxycarbonylamino)-4(S)-hydroxy-6-phenyl-2(R)-4-(2-hydroxyethoxy)phenylmethylhexanoyl]-valine-N-3,6,9,12,15-pentaoxapentadecylamide,

N-(2(R)-hydroxy-1(S)-indanyl)-5(S)-(1,1-dimethylethoxycarbonylamino)-4(S)-hydroxy-6-phenyl2(R)-[4-(1,4,7,10-tetraoxaundecyl)phenyl]methyl hexanamide,

N-(2(R)-(hydroxy-1(S)-indanyl)-5(S)-(1,1-dimethylethoxycarbonylamino)-4(S)-hydroxy-6-phenyl-2(R)-[4-(methoxyethoxyethoxy)phenyl]methylhexanamide,

N-(2(R)-hydroxy-1(S)-indanyl)-5(S)-(1,1-dimethylethoxycarbonylamino)-4(S)-hydroxy-6-phenyl-2(R)-[4-(methoxyethoxy)phenyl]methylhexanamide,

N-(2(R)-hydroxy-1(S)-indanyl)-5(S)-(1,1-dimethylethoxycarbonylamino)-4(S)-hydroxy-6-phenyl-2(R)-[4-(1,4,7,10,13-pentoxatetradecyl)phenyl] methyl hexanamide,

N-(4-(3,4-Dihydro-(1H)-2-benzo[c]thiopyranyl-2,2-dioxide))-5(S)-(1,1-dimethylethoxycarbonylamino)-4(S)-hydroxy-6-phenyl-2(R)-4(3-hydroxypropyl)phenylmethylhexanamide,

N-(2(R)-hydroxy-1(S)-indanyl)-5(S)-(1,1-dimethylethyloxycarbonylamino)-4(S)-hydroxy-2(R)-(5-thia-7-hydroxy-2(E)-hepten-1-yl)-6-phenylhexanamide,

N-(2(R)-hydroxy-1(S)-indanyl)-5(S)-(1,1-dimethylethyloxycarbonylamino)-4(S)-hydroxy-2(R)-(5-thia-8-hydroxy-2(E)-octen-1-yl)-6-phenylhexanamide,

N-(2(R)-hydroxy-1(S)-indanyl)-5(S)-(1,1-dimethylethyloxycarbonylamino)-4(S)-hydroxy-2(R)-4-(4-hydroxy-2-thiabut-1-yl)phenylmethyl-6-phenylhexanamide,

N-(2(R)-hydroxy-1(S)-indanyl)-5(S)-(1,1-dimethylethyloxycarbonylamino)-4(S)-hydroxy-2(R)-(5,8,11,14,17-pentaoxaoctadec-2(E)-en-1-yl)-6-phenylhexanamide,

N-(2(R)-hydroxy-1(S)-indanyl)-5(S)-(1,1-dimethylethyloxycarbonylamino)-4(S)-hydroxy-2(R)-4-(2-hydroxyethylsulfonylmethyl)phenylmethyl-6-phenylhexanamide,

N-(2(R)-hydroxy-1(S)-indanyl)-5(S)-(1,1-dimethylethyloxycarbonylamino)-4(S)-hydroxy-2(R)-4-(2,5,8,11,14-pentaoxapentadec-1-yl)phenylmethyl-6-phenylhexanamide,

N-(2(R)-hydroxy-1(S)-indanyl)-5(S)-(1,1-dimethylethyloxycarbonylamino)-4(S)-hydroxy-2(R)-(4-(2-(hydroxy) ethoxy)phenyl)methyl-6-cyclohexyl-hexanamide,

N-(4-fluoro-2(R)-hydroxy-1(S)-indanyl)-5(S)-(1,1-dimethylethyloxycarbonylamino)-4(S)-hydroxy-2(R)-(4-(2-hydroxyethyloxy)phenylmethyl-6-phenylhexanamide,

N-(1-hydroxy-3-methyl-2-cyclopentyl)-5(S)-(1,1-dimethylethoxycarbonyl)amino-4(S)-hydroxy-6-phenyl-2(R)-[4-(2-hydroxyethoxy)phenyl]methylhexanamide,

N-(1-hydroxy-3-methyl-2-cyclopentyl)-5(S)-(1,1-dimethylethoxycarbonylamino)-4(S)-hydroxy-6-phenyl-2(R)-[4-(2-methoxyethoxy)phenyl]methylhexanamide,

N-(2(R)-hydroxy-1(S)-indanyl)-5(S)-(1,1-dimethylethoxycarbonylamino)-4(S)-hydroxy-6-phenyl-2(R)-[4-(1,4,6,9,12,15,18-heptoxa-5-oxo-nonadecyl)phenyl]methylhexanamide,

N-(2(R)-hydroxy-1(S)-indanyl)-5(S)-(1,1-dimethylethoxycarbonylamino)-4(S)-hydroxy-6-phenyl-2(R)-[4-(6-aza-1,4,9,12,15,18-hexoxa-5-oxo-nonadecyl)phenyl]methylhexanamide,

N-(2(R)-hydroxy-1(S)-indanyl)-5(S)-(1,1-dimethylethoxy carbonylamino)-4(S)-hydroxy-6-phenyl-2(R)-[4-(2-phosphoryloxyethoxy)phenyl]methylhexanamide, or

N-(2(R)-hydroxy-1(S)-indanyl)-5(S)-(1,1-dimethylethoxycarbonylamino)-4(S)-hydroxy-6-phenyl-2(R)-[4-(2-phosphoryloxypropyl)phenyl]methylhexanamide,

N-(2(R)-hydroxy-1(S)-indanyl)-5(S)-(3-oxabutyloxycarbonylamino)-4(S)-hydroxy-2(R)-(4-(2-hydroxy) ethoxyphenyl)methyl-6-phenylhexanamide,

N-(2(R)-hydroxy-1(S)-indanyl)-5(S)-(3,6-dioxaheptyloxycarbonylamino)-4(S)-hydroxy-2(R)-((4-(2-hydroxy)ethoxy)phenyl)methyl-6-phenylhexanamide,

N-(2(R)-hydroxy-1(S)-indanyl)-5(S)-(3,6,9,12-tetraoxa-tridecyloxycarbonylamino)-4(S)-hydroxy-6-phenyl-2(R)-[4-(1,4,7-trioxaoctyl)phenyl]methylhexanamide,

N-(2(R)-hydroxy-1(S)-indanyl)-5(S)-(3,6-dioxaheptyloxycarbonylamino)-4(S)-hydroxy-6-phenyl-2(R)-[4-(1,4-dioxapentyl)phenyl]methylhexanamide,

N-(2(R)-hydroxy-1(S)-indanyl)-5(S)-(3, 6-dioxaheptyloxycarbonylamino)-4(S)-hydroxy-6-phenyl-2(R)-[4-(1,4-7-trioxaoctyl)phenyl]methylhexanamide,

N-(2(R)-hydroxy-1(S)-indanyl)-5(S)-(3,6,9-trioxadecyloxycarbonylamino)-4(S)-hydroxy-6-phenyl-2(R)-[4-(1,4,7-trioxaoctyl)phenyl]methylhexanamide,

N-(2(R)-hydroxy-1(S)-indanyl)-5(S)-(3,6,9,12-tetraoxa-tridecyloxycarbonylamino)-4(S)-hydroxy-6-phenyl-2(R)-[4-(1,4-dioxapentyl)phenyl]methylhexanamide,

N-(2(R)-hydroxy-1(S)-indanyl)-5(S)-(2-(2-methoxyethoxy)ethoxycarbonylamino)-4(S)-hydroxy-2(R)-(4-(3(2-methoxyethoxy)methoxy)propyl)phenylmethyl-6-phenylhexanamide,

N-(2(R)-hydroxy-1(S)-indanyl)-5(S)-(3,6,9-trioxadecyloxycarbonylamino)-4(S)-hydroxy-6-phenyl2(R)-[4-(3,6,9,12-tetraoxatridecyloxy)phenyl] methyl-hexanamide,

N-(2(R)-hydroxy-1(S)-indanyl)-5(S)-(3,6,9,12-tetraoxatridecyloxycarbonylamino)-4(S)-hydroxy-6-phenyl 2(R)-[4-(3,6,9,12-tetraoxatridecyloxy)phenyl]methylhexanamide, or

N-(2-(R)-hydroxy-1(S)-indanyl)-5(S)-(1,1-dimethylethoxycarbonylamino)-4(S)-hydroxy-2(R)-(4-(2-phosphoryloxyethoxy)phenyl)methyl-6-cyclohexylhexanamide,

or pharmaceutically acceptable salts thereof.

Of this list, the preferred compounds are

A:

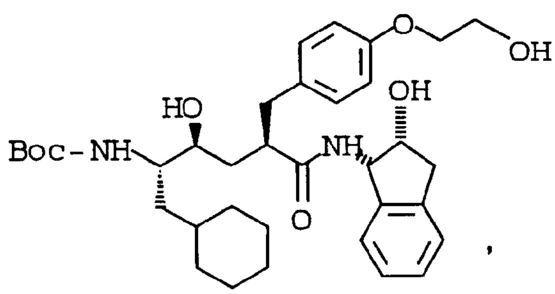

,

N-(2(R)-hydroxy-1(S)-indanyl)-5(S)-(1,1-dimethylethyloxycarbonylamino)-4(S)-hydroxy-2(R)-(4-(2-(hydroxy) ethoxy)phenyl)methyl-6-cyclohexylhexanamide;

B:

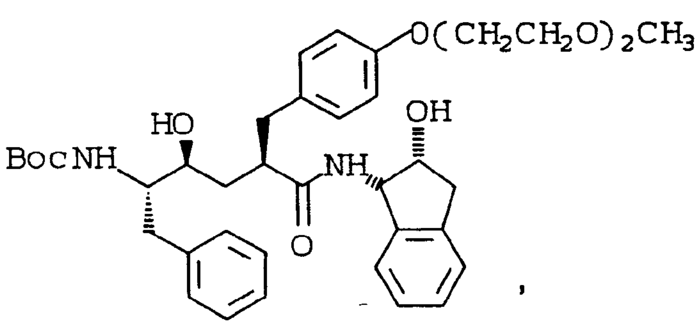

,

N-(2(R)-hydroxy-1(S)-indanyl)-5(S)-(1,1-dimethylethyloxycarbonylamino)-4(S)-hydroxy-2(R)-(4-(1,4,7-trioxa octyl)phenyl)methyl-6-phenylhexanamide;

C:

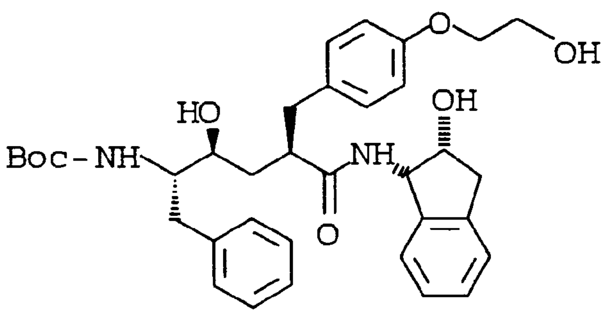

,

N-(2(R)-hydroxy-1(S)-indanyl)-5(S)-(1,1-dimethylethyloxycarbonylamino)-4(S)-hydroxy-2(R)-(4-(2-(hydroxy)ethoxy)phenyl)methyl-6-phenylhexanamide.

D:

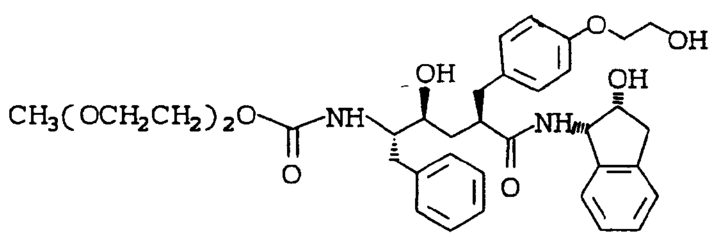

N-(2(R)-hydroxy-1(S)-indanyl)-5(S)-(3,6-dioxaheptyloxycarbonylamino)-4(S)-hydroxy-2(R)-((4-(2-hydroxy)ethoxy)phenyl)methyl-6-phenylhexanamide;

E:

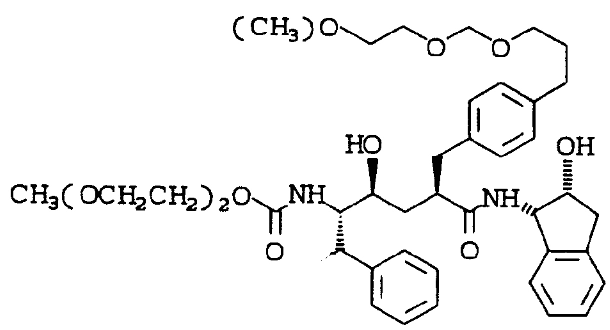

N-(2(R)-hydroxy-1(S)-indanyl)-5(S)-(2-(2-methoxyethoxy)ethoxycarbonylamino)-4(S)-hydroxy-2(R)-(4(3-(2-methoxyethoxy)methoxy)propyl)phenylmethyl-6-phenylhexanamide,

F:

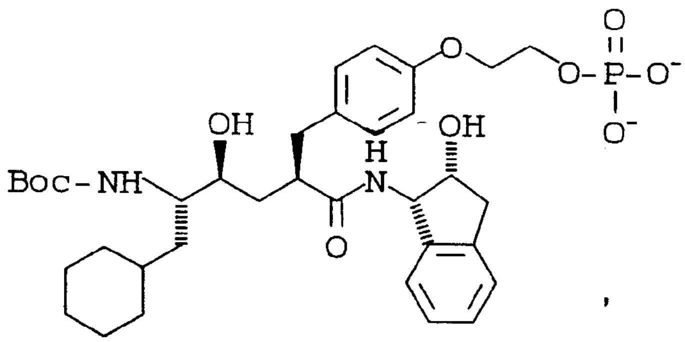

N-(2-(R)-hydroxy-1(S)-indanyl)-5(S)-(1,1-dimethylethoxycarbonylamino)-4(S)-hydroxy-2(R)-(4-(2-phosphory loxyethoxy)phenyl)methyl-6-cyclohexylhexanamide,
or pharmaceutically acceptable salt or ester thereof.

In the compounds of the present invention, the A, G, B and J components and the like may have asymmetric centers and occur as racemates, racemic mixtures and as individual diastereomers, with all isomeric forms being included in the present invention.

when any variable (e.g., aryl, heterocycle, R, $R^9$, $R^9{}_a$, $R^9{}_b$, $R^{10}$, $R^{11}$, $R^{12}$, $R^{15}$, $R^{16}$, $R^{17}$, n, Z, etc.) occurs more than one time in any constituent or in formula I, its definition on each occurrence is independent of its definition at every other occurrence. Also, combinations of substituents and/or variables are permissible only if such combinations result in stable compounds.

As used herein except where noted, "alkyl" is intended to include both branched- and straight-chain saturated aliphatic hydrocarbon groups having the specified number of carbon atoms (Me is methyl, Et is ethyl, Pr is propyl, Bu is butyl); "alkoxy" represents an alkyl group of indicated number of carbon atoms attached through an oxygen bridge. "Alkenyl" is intended to include hydrocarbon claims of either a straight or branched configuration and one or more unsaturated carbon-carbon bonds which may occur in any stable point along the chain, such as ethenyl, propenyl, butenyl, pentenyl, and the like. "Halo", as used herein, means fluoro, chloro, bromo and iodo; and "counterion" is used to represent a small, single negatively-charged species, such as chloride, bromide, hydroxide, acetate, trifluroacetate, perchlorate, nitrate, benzoate, maleate, tartrate, hemitartrate, benzene sulfonate, and the like.

As used herein, with exceptions as noted, "aryl" is intended to mean phenyl (Ph) or naphthyl. "Carbocyclic" is intended to mean any stable 5- to 7-membered carbon ring or 7- to 10-membered bicyclic carbon ring, any of which may be saturated or partially unsaturated.

The term heterocycle or heterocyclic, as used herein except where noted, represents a stable 5- to 7-membered mono- or bicyclic or stable 7- to 10-membered bicyclic heterocyclic ring which is either saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. The heterocyclic ring may be attached at any heteroatom or carbon atom which results in the creation of a stable structure. Examples of such heterocyclic elements include piperidinyl, piperazinyl, 2-oxopiperazinyl, 2-oxopiperidinyl, 2-oxopyrrolodinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazoyl, benzopyranyl, benzothiazolyl, benzoxazolyl, furyl, tetrahydrofuryl, tetrahydropyranyl, thienyl, benzothienyl, thiamorpholinyl, thiamorpholinyl sulfoxide, thiamorpholinyl sulfone, and oxadiazolyl. Morpholino is the same as morpholinyl.

The pharmaceutically-acceptable salts of the compounds of Formula I (in the form of water- or oil-soluble or dispersible products) include the conventional non-toxic salts or the quaternary ammonium salts of these compounds, which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others.

HIV protease inhibitors of Formula I may be prepared in accordance with Schemes I-III. In general, once the G substituent is made, the rest of the synthesis follows the principle of amide bond formation by the coupling methods of either solution-phase or solid-phase peptide synthesis. The addition and removal of one or more protecting groups is also typical practice.

Amide couplings used to form the compounds of this invention are typically performed by the carbodiimide method with reagents such as dicyclohexylcarbodiimide, or 1-ethyl-3-(3-dimethylaminopropyl) carbodiimide. Other methods of forming the amide or peptide bond include, but are not limited to synthetic routes via an acid chloride, azide, mixed anhydride or activated ester. Typically, solution phase amide couplings are performed, but solid-phase synthesis by classical Merrifield techniques may be employed instead.

Some schemes for preparing compounds of formula I are presented below. The examples specifically illustrate the application of the following schemes to specific compounds. Other schemes as well as additional information on synthetic background are found in EPO 0337714; U.S. Patent 4,661,473; Evans, B.E. et al., J. Org. Chem. 50, 4615 (1985); Luly, J.R. et al., J. Org. Chem. 52, 1487 (1987), each document herein incorporated by reference for these purposes.

One scheme for the preparation of the key intermediates involved in this invention is:

SCHEME 1

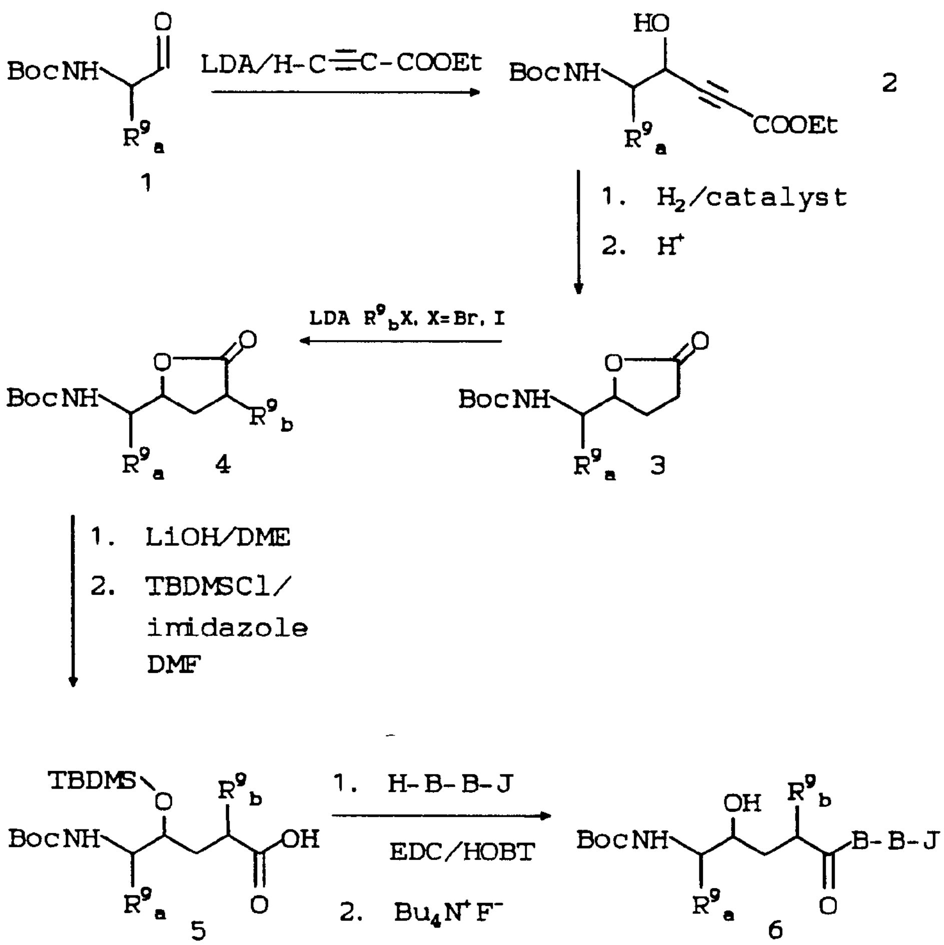


Nucleophilic addition of ethyl propiolate to the aldehyde 1 in the presence of a deprotonating agent (e.g. LDA) yields 2. Hydrogenation in the presence of a palladium, platinum or rhodium catalyst, followed by acid catalysis, yields the lactone 3. A second $R^9$ group is then added by a nucleophilic addition reaction of lactone 3 with a halogenated $R^9$ in the presence of a deprotonating reagent. The resulting $R^9$-substituted lactone 4 can also be synthesized via an epoxide intermediate according to B.E. Evans et al., J. Org. Chem. 50, 4615 (1985), also cited above.

Base hydrolysis of 4 opens the lactone. The gamma-hydroxy acid is then subjected to silylation to prevent relactonization to yield 5. Further coupling by conventional and known techniques, followed by removal of the silyl group (e.g. with $Bu_4N^+F^-$) yields compounds of formula I.

Alternatively, several of the key intermediates in the synthesis of the compounds disclosed in this invention are prepared by the following reaction:

## SCHEME II

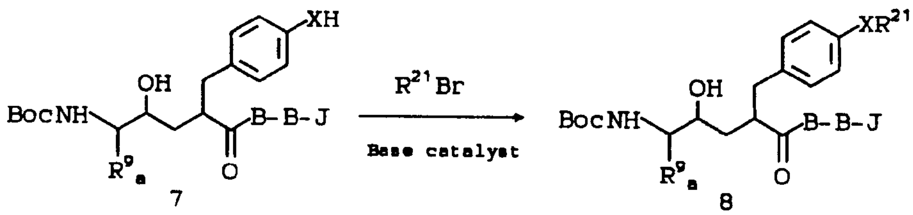


7 8

wherein $R^{21}Br$ is

$Br[((CH_2)_mO)_n]R^{13}$ according to the definition of $R^{12}$, and is representative of an alkylating agent of which in this scheme generates a compound with the substituent $R^9$ in this invention.

Certain carbamates in the current invention are prepared according to Scheme III.

## SCHEME III

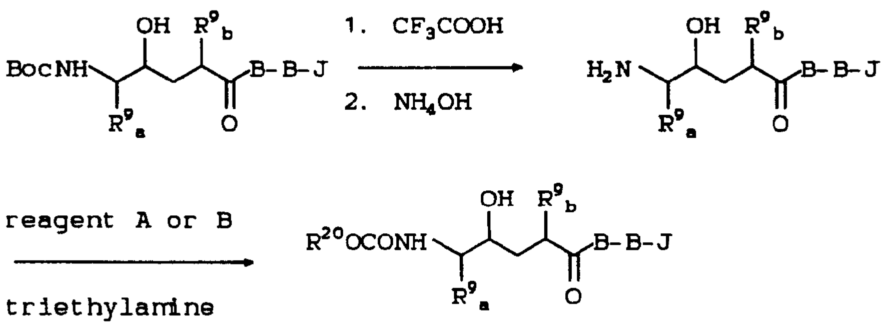


Reagents A and B for generating the carbamates are prepared by the reaction of the appropriate alcohol with p-nitrophenyl chloroformate or sequentially with phosgene (or triphosgene) and N-hydroxy succinimide according to the equations:

1)

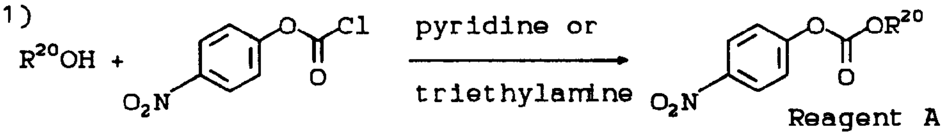


Reagent A

2)

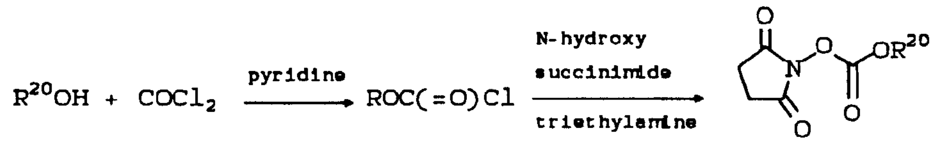


Reagent B

wherein $R^{20}$ encompasses the alcohols contained in the definition of A and which may also be derivatized with an appropriate protecting group such as trialkylsilyl or benzyl.

The compounds of the present invention are useful in the inhibition of HIV protease, the prevention or treatment of infection by the human immunodeficiency virus (HIV) and the treatment of consequent pathological conditions such as AIDS. Treating AIDS or preventing or treating infection by HIV is defined as including, but not limited to, treating a wide range of states of HIV infection: AIDS, ARC (AIDS related complex), both symptomatic and asymptomatic, and actual or potential exposure to HIV. For example, the compounds of this invention are useful in treating infection by HIV after suspected past exposure to HIV by e.g., blood transfusion, accidental needle stick, or exposure to patient blood during surgery.

In the present invention, compounds with asymmetric centers may occur as racemates, racemic mixtures and as individual diastereomers, with all isomeric forms of the compounds being included in the present invention.

For these purposes, the compounds of the present invention may be administered orally, parenterally (including subcutaneous injections, intravenous, intramuscular, intrasternal injection or infusion techniques), by inhalation spray, or rectally, in dosage unit formulations containing conventional non-toxic pharmaceutically-acceptable carriers, adjuvants and vehicles.

Thus, in accordance with the present invention there is further provided a method of treating and a pharmaceutical composition for treating HIV infection and AIDS. The treatment involves administering to a patient in need of such treatment a pharmaceutical composition comprising a pharmaceutical carrier and a therapeutically-effective amount of a compound of the present invention, or a pharmaceutically-acceptable salt thereof.

These pharmaceutical compositions may be in the form of orally-administrable suspensions or tablets; nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweeteners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, flourocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquidify and/or dissolve in the rectal cavity to release the drug.

Dosage levels of the order of 0.02 to 5.0 or 10.0 grams-per-day are useful in the treatment or prevention

of the above-indicated conditions, with oral doses two-to-five times higher. For example, infection by HIV is effectively treated by the administration of from 10 to 50 milligrams of the compound per kilogram of body weight from one to three times per day. It will be understood, however, that the specific dose level and frequency of dosage for any particular patient may be varied and will depend upon a variety of factors including the activity of the specific compound employed, the metabolic stability and length of action of that compound, the age of the patient, body weight, general health, sex, diet, mode and time of administration, rate of excretion, drug combination, the severity of the particular condition, and the host undergoing therapy.

The present invention is also directed to combinations of the HIV protease-inhibitory compounds with one or more agents useful in the treatment of AIDS.

For example, the compounds of this invention can be given in combination with the antivirals, immunomodulaters, antibiotics or vaccines or other derivative forms thereof as listed in the following Table [source: Marketletter, Nov. 30. 1987, pp. 26-27; Genetic Engineering News, Jan. 1988, Vol. 8, 23]:

TABLE[1]

A. Antivirals

| Drug Name | Manufacturer | Indication |
|---|---|---|
| AL-721 | Ethigen | ARC, PGL |
| BETASERON (interferon beta) | Triton Biosciences | AIDS, ARC, KS |
| CARRISYN (polymannoacetate) | Carrington Labs | ARC |
| CYTOVENE (ganciclovir) | Syntex | CMV |
| DDC (dideoxycytidine) | Hoffmann-La Roche | AIDS, ARC |
| ddI (dideoxyinosine) | Bristol-Myers | AIDS, ARC |

[1]Abbreviations: AIDS (Acquired Immune Deficiency Syndrome); ARC (AIDS related complex); CMV (Cytomegalovirus, which causes an opportunistic infection resulting in blindness or death in AIDS patients); HIV (Human Immunodeficiency Virus, previously known as LAV, HTLV-III or ARV); KS (Kaposi's sarcoma); PCP (Pneumonocystis carinii pneumonia, an opportunistic infection); PGL (persistent generalized lymphadenopathy).

| Drug Name | Manufacturer | Indication |
| --- | --- | --- |
| FOSCARNET (trisodium phosphonoformate) | Astra AB | HIV inf, CMV retinitis |
| HPA-23 | Rhone-Poulenc Sante | HIV infection |
| ORNIDYL (eflornithine) | Merrell Dow | PCP |
| PEPTIDE T (octapeptide sequence) | Peninsula Labs | AIDS |
| RETICULOSE (nucleophospho-protein) | Advanced Viral Research | AIDS, ARC |
| RETROVIR advanced (zidovudine; AZT) | Burroughs Wellcome | AIDS, ARC pediatric AIDS, KS, asympt HIV, less severe HIV, neurological involvement. |

| Drug Name | Manufacturer | Indication |
|---|---|---|
| RIFABUTIN (ansamycin LM 427) | Adria Labs | ARC |
| (trimetrexate) | Warner-Lambert | PCP |
| UA001 | Ueno Fine Chem Industry | AIDS, ARC |
| VIRAZOLE (ribavirin) | Viratek/ICN | AIDS, ARC, KS |
| WELLFERON (alfa interferon) | Burroughs Wellcome | KS, HIV, in comb with AZT |
| ZOVIRAX (acyclovir) | Burroughs Wellcome | AIDS, ARC, in comb with AZT |

B. Immunomodulators

| Drug Name | Manufacturer | Indication |
|---|---|---|
| ABPP KS (bropirimine) | Upjohn | Advanced AIDS, |
| AMPLIGEN (mismatched RNA) | DuPont HEM Research | ARC, PGL |
| (Anti-human alpha interferon antibody) | Advanced Biotherapy Concepts | AIDS, ARC, KS |

| | | |
|---|---|---|
| Colony Stimulating Factor (GM-CSF) | Sandoz Genetics Institute | AIDS, ARC, HIV, KS |
| CL246,738 (CL246,738) | American Cynamid | AIDS |
| IMREG-1 | Imreg | AIDS, ARC, PGL, KS |
| IMREG-2 | Imreg | AIDS, ARC, PGL, KS |
| IMUTHIOL (diethyl dithio carbamate) | Merieux Institute | AIDS, ARC |

| Drug Name | Manufacturer | Indication |
|---|---|---|
| IL-2 (interleukin-2) | Cetus | AIDS, KS |
| IL-2 (interleukin-2) | Hoffmann-La Roche Immunex | AIDS, KS |
| INTRON-A (interferon alfa) | Schering-Plough | KS |
| ISOPRINOSINE (inosine pranobex) | Newport Pharmaceuticals | ARC, PGL, HIV seropositive patients |
| (methionine enkephalin) | TNI Pharmaceuticals | AIDS, ARC |

| | | |
|---|---|---|
| MTP-PE (muramyl-tripeptide) | Ciba-Geigy | KS |
| THYMOPENTIN (TP-5) (thymic compound) | Ortho Pharmaceuticals | HIV infection |
| ROFERON (interferon alfa) | Hoffmann-La Roche | KS |
| (recombinant erythropoietin) | Ortho Pharmaceuticals | severe anemia assoc with AIDS & AZT therapy |
| TREXAN (naltrexone) | DuPont | AIDS, ARC |
| TNF (tumor necrosis factor) | Genentech | ARC, in combination interferon gamma |

C. Antibiotics

| Drug Name | Manufacturer | Indication |
|---|---|---|
| PENTAM 300 (pentamidine isethionate) | LyphoMed | PCP |

D. Vaccines

Any one of a variety of AIDS or HIV vaccines presently under study and development can be used in combination with the compounds of this invention or salt or derivative forms thereof, in the treatment or prevention of AIDS and diseases of similar character caused by HIV.

It will be understood that the scope of combinations of the compounds of this invention with AIDS antivirals, immunomodulators, antibiotics or vaccines is not limited to the list in the above Table, but includes in principle any combination with any pharmaceutical composition useful for the treatment of AIDS.

EXAMPLE 1

Preparation of N-(2(R)-hydroxy-1(S)-indanyl)-5(S)-(1,1-dimethylethoxycarbonylamino)-4(S)-hydroxy-2(R)((4-(2-hydroxy)ethoxyphenyl)methyl-6-phenylhexanamide

Step A: 4-Allyloxybenzylbromide

This compound was synthesized from the known 4-allyloxy benzylalcohol using triphenyl phosphine and tetrabromomethane in methylene chloride. The title compound was purified by distillation with a boiling point of 78-82°C at 0.1 mm Hg. NMR ($CDCl_3$): δ 4.49 (s, 2H); 4.52 (t, 1H); 4.54 (t, 1H); 5.29 (m, 1H); 5.40 (m, 1H); 6.04 (m, 1H); 6.88 (m, 2H); 7.30 (m, 2H).

Step B: 5(S)-[1′(S)-(1,1-dimethylethoxycarbonylamino)-2-phenylethyl]-3(R)-4-allyloxyphenylmethyldihydrofuran-2-(3H)-one

A lithium diisopropylamide solution was prepared by dissolving diisopropylamine (1.85 ml) in tetrahydrofuran (20 ml), cooling the solution to -10°C, and slowly adding 6.6 ml of a hexane solution of n-butyl lithium. The lithium diisopropylamide solution was added dropwise to a cold (-78°C) solution of 5(S)-[1′(S)-(1,1-dimethylethoxycarbonylamino)-2-phenylethyl]-dihydrofuran-2-(3H)-one (2 g) in tetrahydrofuran. When addition was complete, the reaction mixture was allowed to warm to -56°C over one hour and then it was cooled back to -78°C. A solution of 4-allyloxybenzylbromide (1.47 g) in tetrahydrofuran (20 ml) was added dropwise to the reaction mixture at a rate such that the temperature of the reaction mixture did not exceed -75°C. After stirring at -78°C for 1 1/2 hours, the reaction mixture was raised in temperature to -57°C for 1 hour and then quenched by addition of acetic acid in tetrahydrofuran. The reaction mixture was filtered of solids and the filtrate was concentrated in vacuo. The residue was partitioned between ether and 10% citric acid solution. The organic phase was separated, washed with brine, dried ($MgSO_4$), filtered and concentrated in vacuo to give a solid plus oil residue. This was chromatographed to yield 350 mg of the title compound as an oil.

Step C: 5(S)-(1,1-dimethylethoxycarbonylamino)-4(S)t-butyldimethylsilyloxy-6-phenyl-2(R)-4-allyloxyphenylmethyl hexanoic acid

5(S)-[1′(S)-(1,1-dimethylethoxycarbonylamino)-2-phenylethyl]-3(R)-4-allyloxyphenylmethyldihydrofuran -2-(3H)-one (349 mg) was dissolved in dimethoxyethane (5 ml) and IN lithium hydroxide (1.5 ml) was added. After 30 minutes 10% citric acid solution (1.5 ml) was added and the reaction mixture was concentrated in vacuo . The solid residue was partitioned between water and ethyl acetate. The organic phase was separated, washed with brine, dried ($MgSO_4$), filtered and concentrated in vacuo to a white solid. This solid was dissolved in anhydrous dimethylformamide (2 ml) along with imidazole (525 mg) and tert-butyldimethylsilyl chloride. The reaction mixture was stirred at room temperature under an argon atmosphere for 16 hours. Water (56 μl was added to the reaction mixture and after 2 hours of stirring, the reaction mixture was concentrated in vacuo. The residue was partitioned between ethyl acetate and 10% citric acid solution. The organic phase was separated, washed with brine, dried ($MgSO_4$), filtered and concentrated in vacuo to an oil residue. The oil residue was chromatographed to yield 302 mg of the title compound as a glassy solid.

Step D: N-(2(R)-hydroxy-1(S)-indanyl)-5(S)-(1,1-dimethylethoxycarbonylamino)-4(S)-t-butyldimethylsilyloxy-6-phenyl-2(R)-4-allyloxyphenylmethyl-hexanamide

5(S)-(1,1-dimethylethoxycarbonylamino)-4(S)-t-butyldimethylsilyloxy-6-phenyl-2(R)-4-allyloxyphenylmethyl hexanoic acid (578 mg), 1-hydroxybenzotriazole (147 mg) and N-ethyl-N′-(3-dimethylaminopropyl)carbodiimidehydrochloride (214 mg) were dissolved together in 3 ml of anhydrous dimethylformamide under an atomosphere of argon. After stirring 5 minutes, 1(S)-amino-2(R)-hydroxyindane (200 mg) was added followed by addition of triethylamine (310 μl). This reaction mixture was stirred for 16 hours at room temperature, concentrated in vacuo, and the residue partitioned between ethyl acetate and 10% citric acid solution. The organic phase was separated, washed with brine, dried ($MgSO_4$), filtered and concentrated in vacuo to yield 679 mg of the title compound as a foamy white solid.

Step E: N-(2(R)-hydroxy-1(S)-indanyl)-5(S)-(1,1-dimethylethoxycarbonylamino)-4(S)-t-butyldimethylsilyloxy-6-phenyl-2(R)-[4-(2-hydroxyethoxy)phenyl]methyl hexanamide

N-(2(R)-hydroxy-1(S)-indanyl)-5(S)-(1,1-dimethylethoxycarbonylamino)-4(S)-t-butyldimethylsilyloxy-6-phenyl-2(R)-4-allyloxyphenylmethyl hexanamide (256 mg) was dissolved in a mixture of methylene chloride (5 ml) and methanol (5 ml) and the solution was cooled to -78°C. Ozone was blown into the solution until a faint blue color persisted. Excess ozone was blown out of the reaction vessel by bubbling argon into the solution. Sodium borohydride (40 mg) was then added and the reaction mixture was allowed to warm to room temperature. The reaction mixture was concentrated in vacuo and the solid residue partitioned between ethyl acetate and water. The organic phase was separated, dried ($MgSO_4$), filtered and concentrated in vacuo.

Step F: N-(2(R)-hydroxy-1(S)-indanyl)-5(S)-(1,1-dimethylethoxycarbonylamino)-4(S)-hydroxy-6-phenyl-2(R)-[4-(2-hydroxyethoxy)phenyl]methylhexanamide

The solid product of Step E was dissolved in a one molar solution of tetrabutylammonium fluoride in tetrahydrofuran and stirred for 2 days at room temperature. The reaction mixture was then poured into water and a resulting solid filtered. This solid was chromatographed to yield 165 mg of the title compound as a white crystalline solid m.p. 199.4-199.6.

| | |
|---|---|
| Anal. Calc'd. for $C_{35}H_{44}N_2O_7$: | C, 69.51; H, 7.33; N. 4.63. |
| Found: | C, 69.88; H. 7.34; N. 4.47. |

EXAMPLE 2

N-(cis-2(R)-hydroxy-1(S)-indanyl)-5(S)-1,1-dimethylethoxycarbonyl)amino-4(S)-hydroxy-6-phenyl-2(R)-4-(2-hydroxyethyl)phenylmethylhexanamide

Step A: Preparation of 3(R)-(4-(2-hydroxyethyl)phenylmethyl-5(S)-(1(S)-((1,1-dimethylethoxycarbonyl)amino)-2-phenylethyl)dihydrofuran-2-(3H)-one

To a 100 ml round bottomed flask with a stirring bar and an argon inlet was added (5(S), 1'(S))-3-carboethoxy-5-(1-(1,1-dimethylethoxycarbonyl)amino)-2-phenylethyl)-dihydrofuran-2-(3H)-one (1.427 g, 3.78 mmol), absolute ethanol (10 ml), and sodium ethoxide (0.257 g, 3.78 mmol). This mixture was stirred for 15 minutes then 4-(2-hydroxyethyl)benzyl bromide (0.813 g, 3.78 mmol) was added in one portion. This mixture was stirred at room temperature for 6 hours then water (10 ml) and lithium hydroxide (0.453 g, 18.90 mmol) were added. The resulting solution was stirred at room temperature for 24 hours. The solvents were removed in vacuo and the residue was suspended in 10% aqueous citric acid. This mixture was extracted with several portions of ethyl acetate. The combined ethyl acetate extracts were washed with water and brine. This solution was dried over $MgSO_4$, filtered and concentrated in vacuo. The residue was dissolved in 200 ml of toluene and the resulting solution was heated at reflux for 24 hours. The cooled solution was concentrated in vacuo and the residue was chromatographed on 100 g of silica gel using 2:3 ethyl acetate-hexanes as eluant. The faster eluting isomer (0.486 g) has the desired 3(R) configuration of the title compound.

Step B: Preparation of N-(1,1-dimethylethoxycarbonyl)-5(S)-amino-4(S)-(1',1'-dimethylethyl-1,1-dimethylsilyloxy-6-phenyl-2(R)-(4-(1',1'-dimethylethyl-1,1-dimethylsilyloxyethyl)phenylmethyl)hexanoic acid

To a 100 ml round bottomed flask with a stirring bar and an argon inlet was added product lactone (0.486 g, 1.11 mmol) from Step A, dimethoxyethane (6.5 ml), water (6.5 ml) and lithium hydroxide (0.132 g, 5.53 mmol). This mixture was stirred at room temperature for 3 hours, poured into 10% aqueous citric acid, and extracted with ethyl acetate. The ethyl acetate solution was dried over $MgSO_4$, filtered and concentrated in vacuo. The residue was dissolved in dry DMF (13 ml) and imidazole (1.505 g, 22.11 mmol) and tert-butyldimethylsilyl chloride (1.666 g, 11.06 mmol) were added. This mixture was stirred at room temperature for 20 hours. To this solution was added methanol (30 ml) and the resulting mixture was again stirred at room temperature for 3 hours. The solvents were removed in vacuo and the residue was dissolved in ethyl acetate. This solution was washed with 10% aqueous citric acid, water and brine. Drying ($MgSO_4$), filtration and removal of the solvent in vacuo gave the crude bis-silyloxy hexanoic acid. This material was chromatographed on silica gel using 3:97 methanol-chloroform as eluant. There was obtained 0.249 g of the title compound as a colorless glass.

Step C: Preparation of N-(2(R)-hydroxy-1(S)-indanyl)-5(S)-(1,1-dimethylethoxycarbonyl)amino)-4(S)(1′,1′-dimethylethyl-1,1-dimethylsilyloxy)-6-phenyl-2(R)-(4-(1′,1′-dimethylethyl-1,1-dimethylsilyloxyethyl)phenylmethyl)hexanamide

To a 50 ml round bottomed flask with a stirring bar and an argon inlet was added the product bis-silyloxy acid from Step B (0.248 g, 0.361 mmol), 2(R)-hydroxy-1(S)aminoindane (0.065 g, 0.434 mmol), ethyl dimethylaminopropylcarbodiimide hydrochloride (0.083 g, 0.434 mmol), hydroxybenztriazole hydrate (0.059 g, 0.434 mmol), DMF (3.00 ml) and triethylamine (0.139 ml, 1.00 mmol). The resulting mixture was stirred at room temperature for 18 hours. The reaction mixture was poured into 10% aqueous citric acid and the solution was extracted with two portions of ethyl acetate. The combined ethyl acetate extracts were washed with water and brine. Drying ($MgSO_4$), filtration and removal of the solvent in vacuo gave the crude coupling product. This material was chromatographed on silica gel using 8:92 ethyl acetate-chloroform as eluant. There was obtained 0.152 g of the title compound as a colorless glass.

Step D: Preparation of N-(cis-2(S)-hydroxy-1(R)-indanyl)-5(S)-1,1-dimethylethoxycarbonyl)amino-4(S)-hydroxy-6-phenyl-2(R)-4-(2-hydroxyethyl)phenylmethyl hexanamide

To a 25 ml round bottomed flask with a stirring bar and an argon inlet was added the bis-silyloxy ether from Step C (0.152 g, 0.186 mmol) and a solution of tetra butylammonium fluoride in THF (4.0 ml of a 0.5 M solution). This solution was stirred at room temperature for 19 hours. The reaction mixture was poured into ethyl acetate (150 ml) and this solution was washed successively with 10% aqueous citric acid, saturated aqueous $NaHCO_3$, and brine. Drying ($MgSO_4$), filtration, and removal of the solvent in vacuo gave the crude product as a white solid. This material was recrystallized from boiling ethyl acetate (10 ml). There was obtained 0.075 g of the title compound as finely divided white crystals mp: 221-222°C.

EXAMPLE 3

N-(cis-2(S)-hydroxy-1(R)-indanyl)-5(S)-(1,1-dimethylethoxycarbonyl)amino-4(S)-hydroxy-6-phenyl-2(R)-4-(3-hydroxypropyl)phenylmethylhexanamide

The title compound was prepared in a manner similar to the described for Example 2 substituting 4-(3-hydroxypropyl)benzyl bromide for 4-(2-hydroxyethyl)benzyl bromide in Step A. The title compound has mp: 190-192°C.

EXAMPLE 4

N-(cis-2(R)-hydroxy-1(S)-indanyl)-5(S)-1,1-dimethylethoxycarbonyl)amino-4(S)-hydroxy-6-phenyl-2(R)-(4-(2(2-methoxyethoxy)methoxyethyl)phenylmethylhexanamide

To a 10 ml round bottomed flask with a stirring bar and an argon inlet was added N-(cis-2(R)-hydroxy-1(S)-indanyl)-5(S)-(1,1-dimethylethoxycarbonyl)amino-4(S)-hydroxy-6-phenyl-2(R)-4-(2-hydroxyethyl)phenylmet hylhexanamide (0.080 g, 0.136 mmol), methylene chloride (5.0 ml, diisopropylethylamine (0.044 ml, 0.250 mmol), and 2-methoxyethoxymethyl chloride (0.020 ml, 0.160 mmol). This solution was stirred at room temperature for 72 hours. The reaction was poured into aqueous saturated $NaHCO_3$, and the methylene chloride was removed in vacuo. The residue was extracted with ethyl acetate and the combined ethyl acetate extracts were washed with brine and dried ($MgSO_4$). Filtration and removal of the solvent in vacuo gave a residue which was chromatographed on silica gel using 2:98 methanol-chloroform as eluant. The chromatographed product was further purified by recrystallization from boiling ethyl acetate. There was obtained 0.040 g of the title compound as white crystals mp: 172-173°C.

EXAMPLE 5

N-(2(R)-hydroxy-1(S)-indanyl)-5(S)-(1,1-dimethylethoxycarbonyl)amino-4(S)-hydroxy-6-phenyl-2(R)-(4-(3-(2-methoxyethoxy)methoxypropyl)-phenyl)methylhexanamide

Using the method of Example 4, the title compound (mp 176-177 °C) was prepared from N-(2(R)hydroxy-1(S)-indanyl)-5(S)-(1,1-dimethylethoxycarbonyl)amino-4(S)-hydroxy-6-phenyl-2(R)-(4-(3-hydroxypropyl)phe nyl)methylhexanamide.

EXAMPLE 6

N-[2(R)-hydroxy-1(S)-indanyl]-5(S)-(1,1-dimethylethoxycarbonylamino)-4(S)-hydroxy-6-phenyl-2(R)-[4(1,4,6,9,12,15,18-heptoxa-5-oxononadecyl)phenyl]methyl hexanamide

N-[2(R)-hydroxy-1(S)-indanyl]-5(S)-(1,1-dimethylethoxycarbonylamino)-4(S)-hydroxy-6-phenyl-2(R)-[4-(2-hydroxyethoxy)phenyl]methylhexanamide (75 mg) was reacted with 60 mg of 3,6,9,12-tetroxatridecyl-4-nitrophenyl carbonate and 15.4 mg of 4-dimethylamino pyridine in dimethylformamide (5 ml) at 60°C for 64 hours. Solvent was removed in vacuo and the oil residue was partitioned between ethyl acetate and 10% citric acid solution. The organic phase was separated, washed with 5% ammonium hydroxide solution, then brine, dried ($MgSO_4$), filtered and concentrated in vacuo to give a semisolid residue. The residue was chromatographed to yield 39 mg of the title compound as a white solid, mp 155.0-155.5°C.

EXAMPLE 7

N-[2(R)-hydroxy-1(S)-indanyl]-5(S)-(1,1-dimethylethoxycarbonylamino)-4(S)-hydroxy-6-phenyl-2(R)[4-(6-aza-1,4,9,12,15,18-hexoxa-5-oxononadecyl)phenyl]methyl hexanamide

N-[2(R)-hydroxy-[(S)-indanyl]-5(S)-(1,1-dimethylethoxycarbonylamino)-4(S)-hydroxy-6-phenyl-2(R)-[4-(2-hydroxyethoxy)phenyl]methyl hexanamide (76 mg) was reacted with 4-nitrophenylchloroformate (50 mg) and pyridine (20 µl) in tetrahydrofuran (1 ml). After 2.5 hours 50 mg of 2,5,8,11-tetraoxatridecan-13-amine was added to the reaction mixture. After 64 hours the reaction mixture was concentrated in vacuo and the oil residue chromatographed to give the title compound as a solid weighing 39 mg, mp 160.5-161.2°C.

EXAMPLE 8

N-(2(R)-hydroxy-1(S)-indanyl)-5(S)-(1,1-dimethylethoxycarbonylamino)-4(S)-hydroxy-6-phenyl-2(R)-[4-(1,4,7-trioxaoctyl)phenyl]methylhexanamide

Step A: Preparation of 4-(1,4,7-trioxaoctyl)benzylbromide

The conversion of 4-hydroxy benzyl alcohol to the title compound was achieved by alkylation with 1-bromo-3,6-dioxaheptane (sodium hydride/DMF) followed by reaction with carbon tetrabromide and triphenyl phosphine under conditions well-precedented in the literature.

Step B: N-(2(R)-hydroxy-1(S)-indanyl)-5(S)-(1,1-dimethylethoxycarbonylamino)-4(S)-hydroxy-6-phenyl-2(R)-[4-(1,4,7-trioxaoctyl)phenyl]methylhexanamide

Following the procedure of Example 1, Steps B-D and F, the product of Step A and 5(S)-[1'(S)-(1,1-dimethylethoxycarbonylamino)-2-phenylethyl]dihydrofuran-2-(3H)-one were converted to the title compound. Anal. Calcd for $C_{38}H_{50}N_2O_8$: C, 68.86; H, 7.60; N, 4.23. Found: C, 68.99; H, 7.54; N, 4.58.

EXAMPLE 9

N-(2(R)-hydroxy-1(S)-indanyl)-5(S)-(1,1-dimethylethoxycarbonylamino)-4(S)-hydroxy-6-phenyl-2(R)-[4-(1,4,7,10,13-pentaoxatetradecyl)phenyl]methylhexanamide

To a solution of the known compound N-(2(R)hydroxy-1(S)-indanyl)-5(S)-(1,1-dimethylethoxycarbonylamino)-4(S)-hydroxy-6-phenyl-2(R)-(4-hydroxyphenyl)methylhexanamide (45 mg, .0599 mmol) in 4 mL of anhydrous dioxane was added cesium carbonate (58.5 mg, 0.179 mmol) and 1-iodo-3,6,9,12-tetraoxatridecane (190 mg, 0.599 mmol). The solution was warmed to 80°C for 12 hours, cooled to ambient temperature, diluted with chloroform and filtered through celite. Concentration of the solution afforded a white solid which was recrystallized from ethyl acetate and hexanes to afford 20 mg (44% yield) of alkylated product. m.p. 163-164°C.
$^1H$ NMR (300 MHz) $\delta$ 7.32-7.21 (m, 8H), 7.06 (m, 3H), 6.82 (m, 2H), 6.00 (br d, J=7.6Hz, 1H), 5.23 (dd, J=8.4, 4.8Hz, 1H), 4.91 (br d, J=6.8Hz, 1H), 4.24 (m, 1H), 4 08 (m, 2H), 3.93 (br s, 1H), 3.82 (m, 4H), 3.72-3.59 (m, 8H), 3.54-3.51 (m, 2H), 3.35 (s, 3H), 3.00-2.69 (m, 8H), 1.84-1.80 (m, 2H), 1.45 (d, J=3.6Hz, 1H), 1.38 (br s, 9H); Anal. Calcd for $C_{40}H_{58}N_2O_{10}$: C, 67.18; H, 7.79; N, 3.73. Found: C, 67.02; H, 7.75; N, 3.68.

EXAMPLES 10-11

Using the method of Example 8 or 9, and substituting the appropriate alkylating agents, there were prepared:

N-(2(R)-hydroxy-1(S)-indanyl)-5(S)-(1,1-dimethyl ethoxycarbonylamino)-4(S)-hydroxy-6-phenyl-2(R)-[4-(1,4,7,10-tetraoxaundecyl)phenyl]methyl hexanamide. Anal. Calcd for $C_{40}H_{54}N_2O_9$: C, 67.97; H, 7.70; N, 3.96. Found: C, 67.40; H, 7.51; N, 3.57.

N-(2(R)-hydroxy-1(S)-indanyl)-5(S)-(1,1-dimethyl ethoxycarbonylamino)-4(S)-hydroxy-6-phenyl-2(R)-[4(1,4-dioxapentyl)phenyl]methyl hexanamide. Anal. Calcd for $C_{36}H_{46}N_2O_7$: C, 69.88; H, 7.49; N, 4.53. Found: C, 69.59; H, 7.45; N, 4.46.

EXAMPLE 12

Dilithium N-(2(R)-hydroxy-1(S)-indanyl)-5(S)-(1,1-dimethylethoxycarbonylamino)-4(S)-hydroxy-6-phenyl-2(R)-((4-(2-phosphoryloxy)ethoxy)-phenyl)methyl)hexanoic amide

Step A: Preparation of N-(2(R)-hydroxy-1(S)-indanyl)5(S)-(1,1-dimethylethoxycarbonylamino)-4(S)-hydroxy-6-phenyl-2(R)-[(4-(2-dibenzylphosphoryloxyethoxy)phenyl)methyl]lhexanoic amide

The phosphorylating agent [bis(benzyloxy)(N,N-diisopropylamino)phosphine] used in the following procedure is described in W. Bannwarth and A. Trzeciak, Helv. Chim. Acta., 70 175 (1987).

To a solution of N-(2(R)-hydroxy-1(S)-indanyl)-5(S)-(1,1-dimethylethoxycarbonylamino)-4(S)-hydroxy-6-phenyl-2(R)-((4-(2-hydroxy)ethoxy)phenyl)methyl)hexanoic amide (103 mg, 0.17 mmol) and tetrazole (28 mg, 0.40 mmol) in 2.5 mL THF was added 70 µL (0.21 mmol) of [bis(benzyloxy)-(N,N-diisopropylamino)phosphine]. After 40 minutes, an additional 40 µL of the phosphine and 15 mg tetrazole were added and stirring was continuted for 20 minutes. m-Chloroperoxybenzoic acid (138 mg of 50-60% purity, 0.4 mmol) was added and the mixture was stirred for 1 hour. The mixture was diluted with EtOAc and washed sequentially with 5% $NaHSO_3$, 10% $NaHCO_3$ and brine. After drying, the solvent was evaporated and the residue was chromatographed on silica gel (1-5% $MeOH/CHCl_3$). There was obtained 70 mg of the title compound after crystallization from ethyl acetate/ hexane.

Step B: Dilithium N-(2(R)-hydroxy-1(S)-indanyl)-5(S)-(1,1-dimethylethoxycarbonylamino)-4(S)hydroxy-6-phenyl-2(R)-[(4-(2-phosphoryloxyethyl)phenyl)methyllhexanoic amide

The product of Step A was dissolved in 15 mL 95% ethanol and 35 mg 10% Pd/C was added. This mixture was stirred 1.5 hours under an atmosphere of hydrogen. Lithium hydroxide (165 µL of IN) was added and the mixture was diluted with water and filtered through Celite. The Celite pad was washed with ethanol and water. Most of the solvent was removed on a rotary evaporator and the residue was filtered through a 0.45 µm membrane and lyophilized to afford 60 mg of the title compound as a fluffy white powder m.p. >300°C.

EXAMPLE 13

Dilithium N-(2(R)-hydroxy-1(S)-indanyl)-5(S)-(1,1-dimethylethoxycarbonylamino)-4(S)-hydroxy-6-phenyl-2(R)-[(4-(3-phosphoryloxypropyl)-phenyl)methyl]hexanoic amide

Applying the method of Example 12 to the product of Example 3 afforded the title compound m.p >300°C.

EXAMPLE 14

[5(S)-(1,1-dimethylethoxycarbonyl-amino)-4(S)-hydroxy-6-phenyl-2(R)-(4-(2-hydroxyethoxy)phenyl)methylhexanoyl]-valine-N-3,6,9,12-tetraoxatridecyl amide.

Step A: N-3,6,9,12-tetraoxatridecylvaline amide

A solution of the N-hydroxy succinimide ester of BOC-L-valine in dimethoxyethane was treated with one equivalent of the known 13-amino-2,5,8,11-tetraoxatridecane to afford the title compound as an oil.

Step B: [5(S)-(1,1-dimethylethoxycarbonylamino)-4(S)hydroxy-6-phenyl-2(R)-(4-(2-hydroxyethoxy) phenyl)methylhexanoyl]-valine-N-3,6,9,12-tetraoxatridecyl amide

Using method of Example 1, Step D, substituting the valine derivative prepared in Step A for 2(R)-hydroxy-1(S)-aminoindane, and further transformation by the methods of Example 1, Steps E and F, the title compound was obtained as a solid, mp 142-143.5 °C Anal. Calcd for $C_{40}H_{63}N_3O_{11}$. 0.3 $H_2O$: C, 62.61; H, 8.35; N, 5.48. Found: C, 62.58; H, 8.22; N, 5.43.

EXAMPLE 15

[5(S)-(1,1-dimethylethoxycarbonylamino)-4(S)-hydroxy-6-phenyl-2(R)-(4-(2-hydroxyethoxy)phenyl)methylhexano-yl]-valine-N-3,6,9,12,15-pentaoxapentadecyl amide

Using the method of Example 14, but using 3,6,9,12,15-pentaoxapentadecyl amine in Step A instead of 13-amino-2,5,8,11-tetraoxatridecane, the title compound was obtained as a white solid, mp 133-141 °C. Anal. Calcd for $C_{41}H_{65}N_3O_{12}$: C, 60.65; H, 8.06; N, 5.15. Found: C, 60.84; H, 8.00; N, 5.12.

EXAMPLE 16

N-(2(R)-hydroxy-1(S)-indanyl)-5(S)-(1,1-dimethylethoxycarbonylamino)-4(S)-hydroxy-2(R)-((4-(2-hydroxy)ethoxy)phenyl)methyl-6-cyclohexylhexanamide

Step A: (5S,1′S)-5-(1′-((1,1-dimethylethoxycarbonyl)amino)-2′-cyclohexylethyl)-4,5-dihydrofuran2-(3H)-one

A solution of the known (5S,1′S)-5-(1′-((1,1-dimethylethoxycarbonyl)amino)-2′-phenylethyl)-4,5-dihydrofuran-2-(3H)-one was dissolved in ethyl acetate and rhodium on alumina was added. This mixture was shaken on a Parr apparatus under a hydrogen atmosphere (50 psi) at 50 °C overnight. Filtration and evaporation of the solvent afforded the title compound as a viscous oil, which solidified gradually to a hard glass.

Step B: N-(2(R)-hydroxy-1(S)-indanyl)-5(S)-(1,1-dimethylethoxycarbonylamino)-4(S)-t-butyldimethylsilyloxy-2(R)-(4-benzyloxyphenyl)methyl-6-cyclohexylhexanamide

The product of Step A was converted by the method of Example 1, Steps B-D to the title compound, employing in Step B benzyloxybenzyl bromide in place of allyloxybenzyl bromide.

Step C: N-(2(R)-hydroxy-1(S)-indanyl)-5(S)-(1,1-dimethylethoxycarbonylamino)-4(S)-t-butyldimethylsilyloxy-2(R)-(4-hydroxyphenyl)methyl-6-phenylhexanamide

The product of Step B was dissolved in ethanol with 10% Pd/C and stirred under an atmosphere of hydrogen for 18 h. The mixture was filtered and the solvent evaporated to afford the title compound.

Step D: N-(2(R)-hydroxy-1(S)-indanyl)-5(S)-(1,1-dimethylethoxycarbonylamino)-4(S)-t-butyldimethylsilyloxy-2(R)-(4-(2-hydroxy)ethoxy)phenyl)methyl-6-cyclohexylhexanamide

To a stirred solution of N-(2(R)-hydroxy-1(S)-indanyl)-5(S)-(1,1-dimethylethoxycarbonylamino)-4(S)-t-butyldimethylsilyloxy-2(R)-(4-hydroxyphenyl)methyl-6-cyclohexylhexanamide (800 mg, 1.17 mmol) in 20 mL of dioxane was added cesium carbonate (1.15 g, 3.52 mmol) and the mixture heated to 80 °C under argon. Ethyl bromoacetate (0.195 mL, 1.76 mmol) was added in one portion, and heating continued for 3.5 h. After cooling the mixture to ambient temperature, 100 mL of chloroform was added and the mixture was filtered and the solids washed with three portions of 30 mL of chloroform. Evaporation of the filtrate gave 918 mg of a solid, which was dissolved in 16 mL of DME, and treated with 2.0 M lithium borohydride in THF (2.3 mL, 4.7 mmol) dropwise. After stirring under argon for 1.5 h, the reaction was carefully quenched with 20 mL of 1M aqueous citric acid. The organic solvents were removed at reduced pressure, and the aqueous residue was extracted with two 40 mL portions ethyl acetate. The combined organic layer was washed with water, 10% sodium carbonate, brine, and dried over magnesium sulfate. Removal of the solvents at reduced pressure gave 853 mg (100%) of the title compound as a colorless foam.

Step E: N-(2R)-hydroxy-1(S)-indanyl)-5(S)-(1,1-dimethylethoxycarbonylamino)-4(S)-hydroxy-2(R)-(4-(2-(hydroxy)ethoxy)phenyl)-methyl-6-cyclohexylhexanamide

The silyl ether derived from Step D (2.55 g, 3.52 mmol) was dissolved in 20 mL of 1M TBAF/THF and stirred under Ar for 24 h. An additional 7 mL of the TBAF solution was added, and stirring continued overnight. A further 5 mL of the TBAF solution was added, the reaction stirred for 6h, then warmed to 35 °C for 4 h. After cooling slightly, the reaction mixture was poured into a stirred mixture of 45 mL of 1M citric acid and 150 mL of ice-water. After stirring for 15 min, the precipitate was collected by filtration and washed with water. The solid was dissolved in $CHCl_3$, dried over $Na_2SO_4$ and the solvents removed to give a yellow solid which was crystallized from 1:1 EtOAc-hexanes to afford 1.51 g of the title compound as colorless crystals, mp 167-168 °C. Anal. Calcd for $C_{35}H_{50}N_2O_7$: C, 68.83; H, 8.25; N, 4.59. Found: C, 68.44; H, 8.30; N, 4.61.

EXAMPLE 17

N-(4-fluoro-2(R)-hydroxy-1(S)-indanyl)-5(S)-(1,1-dimethylethoxycarbonylamino)-4(S)-hydroxy-2(R)-(4-(2-hydroxyethoxy)phenyl)methyl-6-phenylhexanamide

Step A: 4-fluoro-2-hydroxy-2,3-dihydro-(1H)-indene-1-one

To a stirred solution of 1.49 g (26.6 mmol) of KOH in 10 mL of methanol at 0 °C was added dropwise a solution of 1.0 g (6.66 mmol) of 4-fluoro-1-indanone in 15 ml of methanol. After stirring in the cold for 1.5 h, 3.22 g (9.99 mmol) of iodobenzenediacetate was added in four portions over a 1.3 h period. The cold bath was removed and stirring continued for 2 h. The solvents were removed at reduced pressure to give a residue which was triturated with 100 mL of 1:1 EtOAc-$Et_2O$ and the solids removed by filtration. The filtrate was evaporated at reduced pressure to give an oil which was dissolved in 15 mL of ethanol and treated with 10 mL of 10% HCl under Ar for 45 min. The solvents were removed at reduced pressure to give a residue which was dissolved in 120 mL of 1:1 EtOAc-$Et_2O$ and washed with sat $NaHCO_3$, brine, dried over $MgSO_4$, treated with activated carbon and the solvents removed at reduced pressure to give an amber oil. This material was chromatographed on 75 g of fine SiO2 using 1:3 EtOAc-hexanes to afford 538 mg (49%) of the hydroxylated product as a solid.

Step B: cis-1-amino-2-hydroxy-4- fluoro-2,3-dihydro(1H)-indene

To a solution of 6.0 g (36.1 mmol) of the product from step A in 80 mL of pyridine was added 3.0 g (36.1 mmol) of methoxylamine and the solution stirred under Ar for 0.5 h. The solvents were removed at reduced pressure, the residue dissolved in 350 mL of EtOAc, washed with 10% HCl, water, sat. $NaHCO_3$, brine, dried over $MgSO_4$, and the solvents removed to give a dark oil. This material was dissolved in 50 mL of dry THF and added dropwise to 97 mL (96.8 mmol) of 1.0 M borane in THF at 0 °C. After the addition, the cold bath was removed and stirring was continued for 2.5 days. The reaction was then heated at 50 °C for 2 h, cooled to room temperature and carefully quenched with 30 mL of methanol. The solvents were removed at reduced pressure, the residue was twice dissolved in 30 mL of methanol and evaporated to give a foam which was chromatographed on 500 g of fine $SiO_2$ using 90:10:1 $CHCl_3$-$CH_3OH$-$NH_4OH$ to provide 1.385 g (23%) of the aminoalcohol as a colorless solid. The product was resolved into its optical isomers using the method of Evans, et al. [J. Org. Chem. 50, 4615 (1985)]) via the phenylalanyl amides. The less polar diastereomer afforded the desired 1(S)-amino-4-fluoro-2(R)-hydroxyindane.

Step C: N-(4-fluoro-2(R)-hydroxy-1(S)-indanyl)-5(S)-(1,1-dimethylethoxycarbonylamino)-4(S)-t-butyldimethylsilyloxy-2(R)-(4-benzyloxyphenyl)methyl-6-phenylhexanamide

1(S)-Amino-4-fluoro-2(R)-hydroxyindane was coupled to 5(S)-(1,1-dimethylethoxycarbonylamino)-4(S)-t-butyldimethylsilyloxy-2(R)-(4-benzyloxyphenyl)methyl-6-phenylhexanoic acid (see EPO 337 714), as described in Example 1, Step D.

Step D: N-(4-fluoro-2(R)-hydroxy-1(S)-indanyl)-5(S)-(1,1-dimethylethoxycarbonylamino)-4(S)-t-butyldimethylsilyloxy-2(R)-(4-hydroxyphenyl)methyl-6-phenylhexanamide

The product of Step C (130 mg) was dissolved in 4 mL ethanol with 30 mg 10% pd/C and stirred under an atmosphere of hydrogen for 18 h. The mixture was filtered and the solvent evaporated to afford 88 mg of the title compound.

Step E: N-(4-fluoro-2(R)-hydroxy-1(S)-indanyl)-5(S)-(1,1-dimethylethoxycarbonylamino)-4(S)-hydroxy-2(R)-(4-hydroxyethoxyphenyl)methyl-6-phenylhexanamide

The product of Step D was converted to the title compound by the method of Example 16 (Steps D and E). This provided a solid which was chromatographed on 10 g fine $SiO_2$ to give a residue which was triturated with EtOAc-hexanes to afford 38 mg of the title compound as a colorless solid, mp 206-209 °C Anal. Calcd for $C_{35}H_{43}FN_2O_7$ . 0.4 $H_2O$: C, 66.74; H, 7.00; N, 4.45. Found: C, 66.74; H, 6.83; N, 4.34.

EXAMPLE 18

N-(2(R)-hydroxy-1(S)-indanyl)-5(S)-(1,1-dimethylethoxycarbonylamino)-4(S)-hydroxy-2(R)-(4-(4-hydroxy-2-thiabut-1-yl)phenyl)methyl-6-phenylhexanamide

Step A: (5S,3R,1′S)-3-(4-bromomethyl)phenyl)methyl-5-(1′-((1,1-dimethylethoxycarbonyl)amino)-2′-phenylethyl)-4,5-dihydrofuran-2-(3H)-one

To a stirred solution of 1.39 mL (9.9 mmol) of diisopropylamine in 8 mL of dry THF under Ar at 0°C was added 6.14 mL (9.82 mmol) of 1.6 M n-butyllithium in hexanes as a slow stream. After stirring at 0°C for 10 min, the flask was cooled to -78°C, and a solution of 1.5 g (4.91 mmol) of 5(S)-(1′(S)-((1,1-dimethylethoxycarbonyl)amino)-2′-phenylethyl)-4,5-dihydrofuran-2-(3H)-one in 8 mL THF was added dropwise and rinsed in with 1 mL of THF. After the addition, the solution was stirred at -78°C for 20 min, and then added dropwise at -78°C, via jacketed addition funnel, to a -78°C solution of 3.89 g (14.7 mmol) of α, α′-dibromo-p-xylene in 24 mL of THF . The solution was stirred for 1.5 h at -78°C, warmed to -50°C for 20 min and then quenched by the rapid addition of 15 mL of 1M citric acid. This mixture was diluted with 15 mL of water and extracted with two portions of ether. The combined organic layers were washed with water, 10% $Na_2CO_3$, brine, dried over $Na_2SO_4$ and the solvents removed to give an oil which was chromatographed on 40 g of fine $SiO_2$ using $CHCl_3$ followed by 1:1 ethyl acetetehexanes. Product containing fractions were rechromatographed on 100 g of fine $SiO_2$ using 1:4 EtOAc-hexanes to give 351 mg of the bromomethylphenyl lactone as a colorless oil.

Step B: (5S,3R,1′S)-3-((4-hydroxy-2-thiabut-1-yl)phenyl)methyl-5-(1′-((1,1-dimethylethoxycarbonyl)amino)-2′-phenylethyl)-4,5-dihydrofuran-2-(3H)-one

To a stirred solution of the product from step A (78 mg, 0.16 mmol) in 1.5 mL of DMF was added 80 μL (1.14 mmol) of mercaptoethanol and 80 μL (0.459 mmol) of diisopropylethylamine. After stirring for 0.5 h, the reaction mixture was poured into 1 M citric acid and extracted with two portions of ethyl acetate. The combined organic layers were washed with water, 10% $Na_2CO_3$, brine, dried over $Na_2SO_4$ and the solvents removed to give an oil which was chromatographed on 5 g of fine $SiO_2$ using 99:1 $CHCl_3$-$CH_3OH$ to give 52 mg (67%) of the thioether as a colorless oil.

Step C: N-(2(R)-hydroxy-1(S)-indanyl)-5(S)-(1,1-dimethylethoxycarbonylamino)-4(S)-hydroxy-2(R)-(4-(4-hydroxy-2-thiabut-1-yl)phenyl)methyl6-phenylhexanamide

The product from step B was converted to the silyl protected acid, coupled to 2(R)-hydroxy-1(S)-aminoindane, and treated with TBAF/THF by the appropriate methods of Example 1 to give a colorless solid which was chromatographed on 5 g of fine $SiO_2$ using 99:1 to 97:3 $CHCl_3$-$CH_3OH$ to afford a solid which was triturated with 1:4 EtOAc-hexanes to provide 31 mg (46%) of the title compound as a colorless solid: mp, 196-198°C. Anal. Calcd for $C_{36}H_{46}N_2O_6S$ . 0.1 $H_2O$: C, 67.92; H, 7.31; N, 4.40. Found: C, 67.70; H, 7.51; N, 4.24.

EXAMPLE 19

N-(2(R)-hydroxy-1(S)-indanyl) 5(S)-(1,1-dimethylethoxy-carbonylamino)-4(S)-hydroxy-2(R)-(4-(2-hydroxyethylsulfonylmethyl)phenyl)methyl-6-phenylhexanamide

Step A: 5(S)-(1,1-dimethylethoxy-carbonylamino)-4(S)-t-butyldimethylsilyloxy-2(R)-(4-(2-hydroxyethylsulfonylmethyl)-phenyl)methyl-6-phenylhexanoicacid

The intermediate silyl protected acid prepared in Example 18 step C (50 mg, 0.068 mmol) was dissolved in 2 mL of ethanol and treated with a solution of 63 mg (0.102 mmol) of potassium peroxymonosulfate in 1 mL

of ethanol for 3 h at room temperature. Solid $NaHCO_3$ was then added to bring the pH to neutrality, followed by the addition of saturated $NaHCO_3$. The reaction mixture was extracted with two portions of ethyl acetate, and the combined organic layers were washed with water, brine, dried over $MgSO_4$ and the solvents removed at reduced pressure to give the sulfone as a colorless oil.

Step B: N-(2(R)-hydroxy-1(S)-indanyl)-5(S)-(1,1-dimethylethoxycarbonyl-amino)-4(S)-hydroxy-2(R)-(4-(2-hydroxyethyl-sulfonylmethyl)phenyl)methyl-6-phenylhexanamide

The product from step A was coupled to 2(R)-hydroxy-1(S)-amino-indane and treated with TBAF/THF by the methods described in Example 1 to give a colorless solid which was chromatographed on 7 g of fine $SiO_2$ using 95:5 $CHCl_3$-$CH_3OH$ to afford 14 mg (30%) of the title compound as a colorless solid: mp 220-223 °C. Anal: Calcd for $C_{36}H_{46}N_2O_8S$ . 0.75 $H_2O$: C, 63.56; H, 7.04; N, 4.11. Found: C, 63.36; H, 6.82; N, 3.87.

EXAMPLE 20

N-(2(R)-hydroxy-1(S)-indanyl)-5(S)-(1,1-dimethylethoxycarbonylamino)-4(S)-hydroxy-2(R)-(4(2,5,8,11,14-pentaoxapentadec-1-yl)phenyl)methyl-6-phenylhexanamide

Step A: (5(S), 3(R), 1′(S))-3-(4-(2,5,8,11,14-pentaoxa pentadec-1-yl)phenyl)methyl-5-(1′-((1,1-dimethylethoxycarbonyl)amino)-2′-phenylethyl)-4,5-dihydrofuran-2-(3H)-one

To a stirred solution of the product from Example 18, Step A (183 mg, 0.375 mmol) in 1.8 mL of ether was added 84 L (0.432 mmol) of tetraethyleneglycol monomethyl ether and 100 mg (0.432 mmol) of silver (I) oxide. After stirring for 4 days at room temperature, the reaction mixture was filtered through 5 g of fine $SiO_2$ using 95:5 to 90:10 $CHCl_3$-$CH_3OH$ as the eluent. The solvents were removed to give 100 mg (43%) of the ether as a colorless oil.

Step B: N-(2(R)-hydroxy-1(S)-indanyl)-5(S)-(1,1-dimethylethoxycarbonylamino)-4(S)-hydroxy-2(R)-(4-(2,5,8,11,14-pentaoxapentadec-1-yl)phenyl) methyl-6-phenylhexanamide

The product from step A was converted to the silyl protected acid, coupled to 2(R)-hydroxy-1(S)-amino-2,3-dihydro-(1H)-indene, and treated with TBAF/THF according to Example 1 to give a colorless solid which was chromatographed on 5 g of fine $SiO_2$ using 92:8 $CHCl_3$-$CH_3OH$ to afford a solid which was triturated with 1:4 EtOAc-hexanes to provide 40 mg (32%) of the title compound as a colorless solid: mp 145-147 °C. Anal. Calcd for $C_{43}H_{60}N_2O_{10}$: C, 67.52; H, 7.91; N, 3.66. Found: C, 67.46, H, 8.11, N, 3.64.

EXAMPLE 21

N-(4-(3,4-Dihydro-(1H)-benzo[c]thio-pyranyl-2,2-dioxidene))-5(S)-(1,1-dimethylethoxycarbonyl-amino)4(S)-hydroxy-6-phenyl-2(R)-(4-(3-hydroxypropyl)phenylmethyl hexanamide

Step A: N-(4-(3,4-dihydro-(1H)-benzo[c]thiopyranyl2,2-dioxide))-5(S)-(1,1-dimethylethoxycarbonylamino)-4(S)-((1′,1′-dimethylethyl-1,1-dimethylsilyloxy)-6-phenyl-2(R)-(4-(3-[1′,1′dimethylethyl-1,1-dimethylsilyloxy]propyl))phenylmethyl hexanamide

N′-(1,1-Dimethylethoxycarbonyl)-5(S)-amino-4(S)-(1′,1′-dimethylethyl-1,1-dimethylsilyloxy)-6-phenyl-2(R)-(4-[1′,1′-dimethylethyl-1,1-dimethylsilyloxypropyl]phenyl)methyl)hexanoic acid (805 mg, 1.15 mmol), an intermediate prepared in Example 3, was dissolved in 5 mL of dry DMF and to the solution were added 255 mg (1.33 mmol) of 1-(3-dimethylaminopropyl)3-ethylcarbodiimide hydrochloride, 305μL (2.76 mmol) of N-methyl morpholine, 180 mg of 1-hydroxybenzotriazole hydrate (1.33 mmol) and 290 mg of the known compound ±4-amino-3,4-dihydro-(1H)-benzo[c]thiopyran hydrochloride. After stirring for 18 hours under an argon atmosphere, the mixture was concentrated to dryness in vacuo and the residue was partitioned between EtOAc and water acidified to pH 3 with 10% citric acid. The layers were separated and the aqueous extracted once more with EtOAc. The combined organic layers were washed with sat'd. aqueous sodium bicarbonate solution, brine, and dried over sodium sulfate. Removal of the solvent left 1.1 g of an oil which was flash chromatographed in a 55mm column, eluting with 4 liters of 2% EtOAc in chloroform, followed by 2 liters each of 3%, 5%, then 10% EtOAc in chloroform. The undesired diastereomer (410 mg) was eluted first, followed by 310 mg of the title compound. Continued elution with 35% EtOAc provided both diastereomers of the compound desilylated at the

primary hydroxyl group (120 mg of the undesired isomer, followed by 65 mg of the isomer corresponding to the title compound.)

Step B: N-(4-(3,4-Dihydro-(1H)-benzo[c]thiopyranyl-2,2-dioxide))-5(S)-(1,1-dimethylethoxycarbonylamino)-4(S)-hydroxy-6-phenyl-2(R)-(4-(3-hydroxypropyl)phenyl)methyl hexanamide

The 310 mg (0.37 mmol) of the title compound from Step A was deprotected by the method of Example 1, Step F. The crude white solid resulting from this step was dissolved in 5mL of methanol and to this solution was added a solution containing 520 mg (0.85 mmol) of potassium peroxy monosulfate in 5 mL of water. After 18 hours stirring at 25°C, the mixture was concentrated at reduced pressure and the residue was extracted with chloroform/sat'd aqueous sodium bicarbonate mixtures. The combined organic layers were washed with brine and dried over sodium sulfate. Removal of the solvent left a gel which was flash chromatographed, elution with 2% methanol in methylene chloride, followed by a gradient from 4% to 7% methanol in methylene chloride. The title compound obtained as a white solid upon trituration with ether, mp 197-199 °C. Anal. Calcd for $C_{36}H_{46}N_2O_7S$: C, 66.44; H, 7.12; N, 4.30. Found: C, 66.13; H, 7.11; N, 4.24

EXAMPLE 22

N-(2(R)-hydroxy-1(S)-indanyl)-5(S)-(1,1-dimethylethoxycarbonylamino)-4(S)-hydroxy-2(R)-(5-thia-7-hydroxy-2(E)-hepten-1-yl)-6-phenylhexanamide

Step A: Preparation of (5S,3R,1′S)-3-(4-bromo-2(E)buten-1-yl)-5-(1′-((1,1-dimethylethoxycarbonyl)amino)-2′-phenylethyl)-4,5-dihydrofuran-2-(3H)-one

To a stirred solution of 1.93 mL (13.75 mmol) of diisopropylamine in 12 mL of dry THF under Ar at 0 °C was added 8.19 mL (13.09 mmol) of 1.6M n-butyllithium in hexanes as a slow stream. After stirring at 0 °C for 10 min, the flask was cooled to -78°C, and a solution of 2.0 g (6.55 mmol) of the lactone in 12 mL THF was added dropwise. After the addition, the solution stirred at -78°C for 20 min, and then added at -78°C via jacketed addition funnel to a -78°C solution of 4.2 g (19.6 mmol) of 1,4-dibromo-2(E)-butene in 24 mL of THF dropwise. The solution was stirred for 0.5 h at -78°C and then quenched by the rapid addition of 16 mL of 1M citric acid. This mixture was diluted with 15 mL of water and extracted with three portions of ether. The combined organic layers were washed with water, 10% $Na_2CO_3$, brine, dried over $Na_2SO_4$ and the solvents removed to give an oil which was chromatographed on 200 g of fine $SiO_2$ using 1:4 ethyl acetate-hexanes. This yielded 1.97 g (69%) of the title compound as a colorless oil.

Step B: (5S,3R,1′S)-3-(5-thia-7-hydroxy-2(E)-hepten-1-yl)-5-(1′-((1,1-dimethylethoxycarbonyl)amino)-2′-phenyl-ethyl)-4,5-dihydrofuran-2-(3H)-one

To a stirred solution of the product from step A (150 mg, 0.342 mmol) in 0.9 mL of DMF at 0°C was added 150 μL (2.14 mmol) of mercaptoethanol and 150 μL (1.07 mmol) of triethylamine. After stirring for 1 h, the reaction mixture was poured into water and extracted with two portions of ethyl acetate. The combined organic layers were washed with water, brine, dried over $Na_2SO_4$ and the solvents removed to give 146 mg (98%) of the thioether as a colorless oil.

Step C: N-(2(R)-hydroxy-1(S)-indanyl)-5(S)-(1,1-dimethylethoxycarbonylamino)-4(S)hydroxy-2(R)-(5-thia-7-hydroxy-2(E)-hepten-1-yl)-6-phenylhexanamide

The product from step B was converted to the title compound by the methods of Example 1, Steps C,D and F. It was obtained as a colorless solid which was crystallized from 3.5 mL of ethyl acetate to give 81 mg (41%) of the title compound as a colorless solid: mp 160-161°C. Anal. Calc'd for $C_{32}H_{44}N_2O_6S$: C, 65.73; H, 7.58; N, 4.79. Found: C, 66.00; H, 7.66; N, 4.81.

EXAMPLE 23

N-(2(R)-hydroxy-1(S)-indanyl)-5(S)-(1,1-dimethylethoxycarbonylamino)-4(S)-hydroxy-2(R)(5-thia-8-hydroxy-2(E)-octen-1-yl)-6-phenylhexanamide

Step A: Preparation of (5S,3R,1′S)-3-(5-thia-8-hydroxy-2(E)-octen-1-yl)-5-(1′-((1,1-dimethylethoxycarbonyl)amino)-2′-phenylethyl)-4,5-dihydrofuran-2-(3H)-one

To a stirred solution of the bromobutenyl lactone prepared in Example 22, Step A (142 mg, 0.324 mmol) in 2.3 mL of DMF was added 150 μL of 3-mercaptopropanol and 150 μL (1.07 mmol) of triethylamine. After stirring overnight, the reaction mixture was heated to 50°C, and 150 μL portions of mercaptopropanol and triethylamine added, and stirred at 50°C overnight. After cooling to room temperature, the reaction mixture was poured into water and extracted with two portions of ethyl acetate. The combined organic layers were washed with water, brine, dried over $Na_2SO_4$ and the solvents removed to give an oil which was chromatographed on 5 g of fine $SiO_2$ using 2:3 EtOAc-hexanes to afford 56 mg of the thioether as a colorless oil.

Step B: N-(2(R)-hydroxy-1(S)-indanyl)-5(S)-(1,1-dimethylethoxycarbonyl-amino)-4(S)-hydroxy-2(R)-(5-thia-8-hydroxy-2(E)-octen-1-yl)-6-phenylhexanamide

The product from step A was converted to the title compound by the methods of Example 1, Steps C,D and F, to give a colorless solid which was chromatographed on 10 g of fine $SiO_2$ using 98:2 $CHCl_3$-$CH_3OH$ to give a residue which was triturated with 1:4 EtOAc-hexanes to provide 35 mg of the title compound as a colorless solid, mp: softened at 141-143°C, melted at 156-158 °C. Anal. Calcd for $C_{33}H_{46}N_2O_6S$: C, 66.19; H, 7.74; N, 4.68. Found: C, 65.85; H, 7.68; N, 4.42.

EXAMPLE 24

N-(2(R)-hydroxy-1(S)-indanyl) 5(S)-(1,1-dimethylethoxycarbonylamino)-4(S)-hydroxy-2(R)(5,8,11,14,17-pentaoxaoctadec-2(E)-en-1-yl)-6-phenylhexanamide

Step A: (5S,3R,1′S)-3-(5,8,11,14,17-pentaoxaoctadec-2(E)-en-1-yl-5-(1′-((1,1-dimethylethoxycarbonyl)-amino)-2′-phenyl-ethyl)-4,5-dihydrofuran-2-(3H)-one

To a stirred solution of the bromobutenyl lactone prepared in Example 22, Step A (224 mg, 0.51 mmol) in 1.7 mL of ether was added 139 mg (0.6 mmol) of silver (I) oxide and 117 μL (0.6 mmol) of tetraethyleneglycol monomethyl ether. After stirring over the weekend, the reaction mixture was filtered through 3.5 g of fine $SiO_2$2 eluting with 95:5 $CHCl_3$-$CH_3OH$ to give 227 mg of the tetraethyleneglycol ether as a colorless oil.

Step B: N-(2(R)-hydroxy-1(S)-indanyl)5(S)-(1,1-dimethylethoxycarbonylamino)-4(S)-hydroxy-2(R)-(5,8,11,14,17-pentaoxaoctadec-2(E)-en-1-yl)-6-phenylhexanamide

The product from step A was converted to the title compound by the methods described in Example 1, Steps C,D and F, to give a colorless solid which was chromatographed on 10 g of fine $SiO_2$ using 96.5:3.5:0.14 EtOAc-$CH_3OH$-$NH_4OH$ to give 60 mg of the title compound as a colorless solid: mp 118-120 °C. Anal. Calc'd for $C_{39}H_{58}N_2O_{10}$. 0.5 $H_2O$: C, 64.71; H, 8.22; N, 3.87. Found: C, 64.79; H, 8.25; N, 3.86.

EXAMPLE 25

N-(1-hydro-3-methyl-2-cyclopentyl)-5(S)-(1,1-dimethylethoxycarbonyl)amino-4(S)-hydroxy-6-phenyl-2(R)-[4-(2-hydroxyethoxy)phenyl]methylhexanamide

Step A: 2-[N-(1′,2′-di-t-butoxy-carbonyl)-hydrazinol-3-methylcyclopentanone

To a 3-neck round-bottom flask equipped with digital thermometer, and stir bar, is added CuCl (0.297 g, 0.124 mol) and anhydrous diethyl ether (10 ml). The mixture was cooled to -60°C under argon and $Bu_3P$ (0.74 ml, 0.124 mol) was added. The yellow solution was cooled to -73°C, and MeMgCl (Aldrich, 3M in THF, 4.2 ml) was added at a rate so that the internal temperature did not exceed -60°C. The cuprate was stirred for 20 min at -78°C, forming a dark mustard colored solution. Cyclopentenone (1.0 ml, 0.0119 mol) was added dropwise

to this solution by syringe, keeping the temperature below -70°C. The reaction was allowed to proceed at -78°C for 1.5 h. Di-tert-butyl azodicarboxylate (Fluka, 2.8739 g, 0.0125 mol) in 13 ml anhydrous THF and was added by canula over 45 min. The resulting dark brown mixture was stirred for 2 h. The reaction was quenched by addition of 1:9 [$NH_4OH$(con): $NH_4Cl$(sat'd)] and allowed to warm to room temperature overnight. The reaction was diluted with $Et_2O$ and the layers were separated. The $Et_2O$ layer was washed with $NH_4Cl$(sat'd), brine, dried over $Na_2SO_4$, filtered, and concentrated. The resulting oil was purified by flash chromatography using 20% EtOAc/hexane as eluent on a 7 cm. column. Recrystallization from a minimal amount of $Et_2O$ in hexanes gave 0.6680 g of a white solid, mp 100-101.5°C. A second crop also was obtained (0.1108 g, mp 100-101°C). Mass spectrum (FAB) m/e 329.2 (M+1).

Step B: 2-[N-(1′,2′-Di-t-butoxy-carbonyl)hydrazino]-3-methylcyclopentanol

2-[N-(1′,2′-Di-t-butoxycarbonyl)hydrazino]3-methyl-cyclopentanone from step A above (1.1g, <=.0034 mol) was dissolved in anhydrous THF under argon and cooled to -78°C. L-Selectride (Aldrich, 1M, 10 ml, 0.01 mol) was added via syringe, and the reaction stirred at -78°C for 4 h, or until TLC (35%EtOAc/Hexanes ) showed no starting material remaining. The reaction was quenched by cautious addition of 10 mL 1N NaOH solution followed by dropwise addition of 10 ml 30% $H_2O_2$. The solution was allowed to warm to room temperature overnight.

The mixture was poured into $Et_2O$ (75ml) and the layers were separated. The aqueous layer was extracted further with $Et_2O$ (2x 50ml). The combined organic layers were washed with brine, dried over $MgSO_4$, filtered, and concentrated to an oil. The oil was purified by flash chromatography with 20% EtOAc/hexanes and the white solid obtained was recrystallized from hot $Et_2O$/hexane to give a white amorphous solid, mp 146°C. Mass spectrum (FAB) m/e 331.2 (M+ 1) .

Step C: 2-amino-3-methyl cyclopentanol

The alcohol obtained in step B (1.494g, 0.0048mol) was dissolved in $CH_2Cl_2$ (5ml) and cooled to -20°C. Trifluoroacetic acid (10 ml, 0.048 mol) was added by syringe, and the resulting brown mixture was stirred overnight at room temperature. The solvents were removed in vacuo, and the brown residual oil was azeotroped several times with toluene, giving 1.7 g of oil. Without purification the crude hydrazino-methyl cyclopentanol (0.5 g) was dissolved in 4:1 EtOH:HOAc (38 ml) and placed in a Parr shaker flask. To this solution was added $PtO_2$ (72 mg) and the dark green mixture was hydrogenated at 55-60 psi for 2.5 h. The solution was filtered through Celite and concentrated. The resulting brown oil was azeotroped with toluene, and the residue purified by flash chromatography with 45:9:1 ($CHCl_3$: MeOH: c $NH_4OH$). The orange solid thus obtained was triturated with $CHCl_3$/$Et_2O$ (1:5) yielding a white amorphous solid, mp 109-116°C. 1H NMR (300MHz, $CDCl_3$) 3.92-3.96 (m,1H), 2.59-2.64 (m,1H), 2.15 (br s, 3H), 1.92 (m,2H), 1.65 (m,2H), 1.12 (m, 1H), 1.04 (d, J=6.6Hz, 3H).

Step D: N-(1-hydroxy-3-methyl-2-cyclopentyl)-5(S)-(1,1-dimethylethoxy-carbonyl)amino-4(S)-t-butyldimethylsilyloxy-6-phenyl-2(R)-(4-benzyloxynphenyl)methyl-hexanamide

The product of Step C was coupled to the known 5(S)-(1,1-dimethylethoxycarbonylamino)-4(S)-t-butyldimethylsilyloxy-2(R)-(4-benzyloxyphenyl)methyl-6-phenylhexanoic acid (see EPO 337, 714), as described in Example 1, Step D. Flash chromatography was performed using 25% EtOAc/hexanes and the fractions corresponding to Rf 0.2 (35% EtOAc/hexanes) were combined to yield 0.50g of a colorless foam.

Step E: N-(1-hydroxy-3-methyl-2-cyclopentyl)-5(S)-(1,1-dimethylethoxy-carbonyl)amino-4(S)-t-butyldimethylsilyloxy-6-phenyl-2(R)-(4-hydroxyphenyl)methylhexanamide

The foam obtained from step D (0.478 g) was dissolved in 20 ml THF. Palladium hydroxide (0.03 g) was added and the reaction stirred under $H_2$ (balloon) overnight. The solution was filtered through a pad of Celite, and evaporated in vacuo to give a white foam (0.4231 g).

Step F: N-(1-hydroxy-3-methyl-2-cyclo-pentyl)-5(S)-(1,1-dimethylethoxycarbonyl)-amino-4(S)-hydroxy-6-phenyl-2(R)-(4-hydroxy-phenyl)methylhexanamide

The product of Step E (0.4178 g) was dissolved in 1.75 ml 1M tetrabutylammonium fluoride in THF and stirred under argon 24 h. The mixture was concentrated in vacuo, and the residue was chromatographed using 5% MeOH/$CHCl_3$ yielding 0.27 g of a white solid, mp 206-208°C.

Step G: N-(1-hydroxy-3-methyl-2-cyclopentyl)-5(S)-(1,1-dimethylethoxycarbonyl)-amino-4(S)-hydroxy-6-phenyl-2(R)-[4-(2-hydroxyethoxy)phenyl]methylhexanamide

The product of Step F was converted to the title compound by the method of Example 16, Step C. The product was purified by chromatography in 8%MeOH/$CHCl_3$ to give 78 mg of a solid which was triturated with hot EtOAc/hexanes to give a white amorphous solid, mp 163-165°C.

EXAMPLE 26

N-(1-hydroxy-3-methyl-2-cyclopentyl)-5(S)-(1,1-dimethyl ethoxy carbonylamino)-4(S)-hydroxy-hydroxy-6-phenyl-2(R)-[4-(2-methoxyethoxy)phenyl]-ethyl hexanamide

The product obtained from Example 25, step F (0.05 g) was dissolved in dry dioxane (8 mL) with 1-methoxy-2-iodoethane (freshly distilled, 0.217g) and $CsCO_3$ (0.102g). The heterogeneous reaction mixture was stirred at 80°C for 20 h under argon. The reaction was cooled to room temperature, diluted with $CHCl_3$, and filtered through celite. The filtrate was concentrated to give a white solid which was partially purified by column chromatography (5%MeOH/$CHCl_3$). The desired product was further purified by preparative HPLC and trituration with 1:1 hot EtOAc/hexanes, producing a white amorphous solid, mp 183-185°C.

EXAMPLE 27

Preparation of N-(2(R)-hydroxy-1(S)-indanyl)-5(S)-(3-oxabutyloxycarbonylamino)-4(S)-hydroxy-2(R)-((4-(2-hydroxyethoxy)phenyl)methyl-6-phenylhexanamide

Step A: Preparation of N-(2(R)-hydroxy-1(S)-indanyl)-5(S)-amino-4(S)-hydroxy-2(R)-((4-(2-hydroxyethoxy)phenyl)methyl-6-phenylhexanamide

The product of Example 1 (212 mg) was suspended in 15 mL methylene chloride at 0°C. Trifluoroacetic acid (3 mL) was added and the mixture was stirred for 70 minutes at the same temperature. The solvents were removed in vacuo and the residue was treated with saturated sodium bicarbonate. The resulting solid was isolated by filtration and redissolved in methanol. The resulting solution was evaporated to afford 136 mg of the title compound.

Step B: Preparation of N-(2-(R)-hydroxy-1(S)-indanyl-5(S)-(3-oxabutyloxycarbonylamino)-4(S)-hydroxy-2(R)-((4-(2-hydroxyethoxy)phenyl)-methyl-6-phenylhexanamide

Ethylene glycol monomethyl ether (3.5 eq) was dissolved in THF with 2.2 mL (27 mmol) pyridine and 3.0 eq p-nitrophenylchloroformate (2.0 g, 27 mmol) was added. When the exothemic reaction had subsided, the mixture was filtered and evaporated. The residue was dissolved in ether and washed sequentially with 10% citric acid, 10% sodium bicarbonate and brine. The solution was dried and evaporated to an oil which solidified upon trituration with hexane. This solid contained some unreacted p-nitrophenylchloroformate and was resubjected to the reaction conditions above, using triethylamine rather than pyridine catalysis, to completely consume that reagent. A 128 mg (0.52 mmol) sample of the pure mixed carbonate thus obtained was added to a solution in THF/DMF of 66 mg (0.13 mmol) of the product of Step A. After stirring overnight, the solvents were evaporated and the residue was redissolved in ethyl acetate. This solution was washed with 5% ammonium hydroxide and brine. The solution was dried and evaporated to a yellow solid, which was chromatographed on silica gel (methanol/chloroform) to provide the pure title compound.

| | |
|---|---|
| Analysis Calc. for $C_{34}H_{42}N_2O_8$: | C, 67.31; H, 6.98; N, 4.62 |
| Found: | c, 67.51; H, 6.63; N, 4.49 |

EXAMPLE 28

Preparation of N-(2(R)-hydroxy-1(S)-indanyl)-5(S)-(3,6-dioxaheptyloxycarbonylamino)-4(S)-hydroxy-2(R)-((4-(2-hydroxy)ethoxyphenyl)methyl-6-phenylhexanamide

The title compound was prepared according to the method of Example 27 except using diethylene glycol monomethyl ether in Step B.

Analysis. Calc. for $C_{36}H_{46}N_2O_9$: C, 66.44; H, 7.12; N, 4.30.

Found: C, 66.17; H, 6.97; N, 4.27.

EXAMPLE 29

Preparation of N-(2(R)-hydroxy-1(S)-indanyl)-5(S)-(3,6,9,12-tetraoxatridecyloxycarbonylamino)-4(S)-hydroxy-6-phenyl-2(R)-[4-(1,4,7-trioxaoctyl)phenyl]methyl hexanamide

Step A: Preparation of 4-(1,4,7-trioxaoctyl)benzylbromide

The conversion of 4-hydroxy benzyl alcohol to the title compound was achieved by alkylation with 1-bromo-3,6-dioxaheptane (sodium hydride/DMF) followed by reaction with carbon tetrabromide and triphenylphosphine under conventional conditions.

Step B: N-(2(R)-hydroxy-1(S)-indanyl)-5(S)-(1,1-dimethylethoxycarbonylamino)-4(S)-hydroxy-6-phenyl-2(R)-[4(1,4,7-trioxaoctyl)phenyl]methylhexanamide

Following the procedure of Example 1, Steps B-D and F, the product of Step A and 5(S)-[1'(S)-(1,1-dimethylethoxycarbonylamino)-2-phenylethyl]dihydrofuran-2(3H)-one were converted to the title compound. Anal. Calcd for $C_{38}H_{50}N_2O_8$:

C, 68.86; H, 7.60; N, 4.23.
Found: C, 68.99; H, 7.54; N, 4.58.

Step C: N-(2(R)-hydroxy-1(S)-indanyl-5(S)-(3,6,9,12-tetraoxatridecyloxycarbonylamino)-4(S)-hydroxy-6-phenyl-2(R)-[4-(1,4,7-trioxaoctyl)phenyl]methyl hexanamide

Using the method of Example 27, except substituting tetraethylene glycol monomethylether for the ethylene glycol monomethyl ether used therein, the product of Step B was converted to the title compound as a hemihydrate. Anal. Calcd for $C_{43}H_{60}N_2O_{12}\bullet 0.5H_2O$:

C, 65.41; H, 7.50; N, 3.72.
Found: C, 65.43; H, 7.66; N, 3.63.

EXAMPLES 30-33

Using the methods of Example 29, and substituting the appropriate alcohols and alkylating agents, there were prepared:

N-(2(R)-hydroxy-1(S)-indanyl)-5(S)-(3,6-dioxaheptyloxycarbonylamino)-4(S)-hydroxy-6-phenyl-2(R)-[4-(1,4-dioxapentyl)phenyl]methyl hexanamide. Anal. Calcd for $C_{37}H_{48}N_2O_9$:

C, 66.85; H, 7.28; N, 4.21.
Found: C, 67.22; H, 7.25; N, 4.11.

N-(2(R)-hydroxy-1(S)-indanyl)-5(S)-(3,6-dioxaheptyloxycarbonylamino)-4(S)-hydroxy-6-phenyl-2(R)-[4-(1,4,7-trioxaoctyl)phenyl]methyl hexanamide. Anal. Calcd for $C_{39}H_{52}N_2O_{10}$:

C, 66.08; H, 7.39; N, 3.95.
Found: C, 65.69; H, 7.41; N, 3.81.

N-(2(R)-hydroxy-1(S)-indanyl)-5(S)-(3,6,9-trioxadecyloxycarbonylamino)-4(S)-hydroxy-6-phenyl-2(R)-[4-(1,4,7-trioxaoctyl)phenyl]methyl hexanamide. Anal. Calcd for $C_{41}H_{56}N_2O_{11}$:

C, 65.41; H, 7.50; N, 3.72.
Found: C, 65.43; H, 7.66; N, 3.63.

N-(2(R)-hydroxy-1(S)-indanyl)-5(S)-(3,6,9,12-tetraoxatridecyloxycarbonylamino)-4(S)-hydroxy-6-phenyl-2(R)-[4-(1,4)-dioxapentyl)phenyl]methyl hexanamide (0.32) hydrate. Anal. Calcd for $C_{41}H_{56}N_2O_{11}$•0.32 $H_2O$:

C, 64.91; H, 7.53; N, 3.69.
Found: C, 64.90; H, 7.33; N, 3.61.

EXAMPLE 34

N-(cis-2(R)-hydroxy-1(S)-indanyl)-5(S)-(2-(2-methoxyethoxy)ethoxycarbonyl)amino-4(S)-hydroxy-6-phenyl-2(R)-(4-(3-(2-methoxyethoxy)methoxy)propyl)phenylmethylhexanamide

To a 50 ml round bottomed flask with a stirring bar and an argon inlet was added N-(cis-2(R)-hydroxy-1(S)-indanyl)-5(S)-(2-(2-methoxyethoxy)ethoxycarbonyl)amino-4(S)-hydroxy-6-phenyl-2(R)-(4-(3-hydroxypropyl) phenylmethylhexanamide (0.180g, 0.277 mmol), methylene chloride (5.0 ml), diisopropylethylamine (0.116 ml, 0.666 mmol), and 2-methoxyethoxymethyl chloride (0.038 ml, 0.333 mmol). This mixture was stirred at room temperature for 20 hours. An additional portion of 2-methoxyethoxymethyl chloride (0.020 ml) was added and the mixture was stirred for an additional 7 hours. The reaction was quenched by addition of saturated aqueous $NaHCO_3$, and the solvents were removed in vacuo. The aqueous residue was extracted with ethyl acetate and the combined ethyl acetate extracts were washed with 10% aqueous citric acid, water and brine. Drying ($MgSO_4$), filtration and removal of the solvent in vacuo gave the crude product as a crystalline solid. This material was chromatographed on silica gel using 1:4 ethyl acetate-hexanes as eluant. The chromatographed product was further purified by recrystallization from boiling ethyl acetate-hexanes. There was obtained 0.110g of the title compound as a microcrystalline solid m.p. 121-122°C.

EXAMPLES 35-36

Employing the methods of Example 29 with the appropriate modifications there were prepared: N-(2(R)-hydroxy-1(S)-indanyl)-5(S)-(3,6,9-trioxadecyloxycarbonylamino)-4(S)-hydroxy-6-phenyl-2(R)-[4-(3,6,9,12-tet raoxatridecyloxy)phenyl]methyl hexanamide. Anal. Calcd for $C_{45}H_{65}N_2O_{13}$•0.50$H_20$:

C, 63.59; H, 7.71; N, 3.30.
Found: C, 63.59; H, 7.84; N, 3.30.

N-(2(R)-hydroxy-1(S)-indanyl)-5(S)-(3,6,9,12-tetraoxatridecyloxycarbonylamino)-4(S)-hydroxy-6-phenyl-2(R)-[4-(3,6,9,12-tetraoxatridecyloxy)phenyl]methyl hexanamide. Anal. Calcd for $C_{47}H_{68}N_2O_{14}$•0.52$H_2O$:

C, 63.12; H, 7.78; N, 3.15.
Found: C, 63.12; H, 7.92; N, 3.11.

EXAMPLE 37

Preparation of N-(2-(R)-hydroxy-1(S)-indanyl)-5(S)-(1,1-dimethylethoxycarbonylamino)-4(S)-hydroxy-2(R)-(4-(2-phosphoryloxyethoxy)phenyl)methyl-6-cyclohexylhexanamide

The title compound was prepared by applying the method of Example 12 to the product of Example 16. Analysis. Calc. for $C_{35}H_{49}Li_2N_2O_{10}P$•2$H_2O$:

C, 56.91; H, 7.23; N, 3.79
Found: C, 56.56; H, 6.98; N, 3.86

EXAMPLE 38

HIV PROTEASE INHIBITION ASSAY

Inhibition studies of the reaction of the protease expressed in Escherichia coli with a peptide substrate [Val-Ser-Gln-Asn-(betanapthyl)Ala-Pro-Ile-Val, 0.5 mg/mL at the time the reaction is inititated] were in 50 mM Na acetate, pH 5.5, at 30°C for 1 hour. Various concentrations of inhibitor in 1.0 µl DMSO were added to 25 µl of the peptide solution in water. The reaction is initiated by the addition of 15 µl of 0.33 nM protease (0.11 ng) in a solution of 0.133 M Na acetate pH 5.5 and 0.26% bovine serum albumin. The reaction was quenched with 160 µl of 5% phosphoric acid. Products of the reaction were separated by HPLC (VYDAC wide pore 5 cm C-18 reverse phase, acetonitrile gradient, 0.1% phosphoric acid). The extent of inhibition of the reaction was determined from the peak heights of the products. HPLC of the products, independently synthesized, proved quantitation standards and confirmation of the product composition. Compounds of this invention showed $IC_{50}$ values of between about 10 pM and about 10 nM.

While the foregoing specification teaches the principles of the present invention, with examples provided for the purpose of illustration, it will be understood that the practice of the invention encompasses all of the usual variations, adaptations, modifications, deletions or additions as come within the scope of the following claims and its equivalents.

## Claims

**1.** A compound of formula I having the structure:

A-G-B-B-J I,

wherein A is

1) hydrogen;

2) 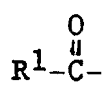

wherein $R^1$ is

a) $C_{1-6}$ alkyl either unsubstituted or substituted with one or more of

i) $C_{1-4}$ alkyl;

ii) hydroxy;

iii) carboxy;

iv) halo wherein halo is F, Cl, Br, or I:

v) amino;

vi) $C_{1-3}$ alkoxycarbonyl;

vii) $C_{1-3}$ alkoxy;

viii) $-CONR^2R^3$ wherein $R^2$ and $R^3$ are the same or different and are hydrogen, $C_{1-5}$ alkyl or $C_{1-5}$ alkoxyalkyl or joined together either directly to form a 5-7 membered heterocycle, or through a heteroatom selected from N, O, and S, to form a 6-membered heterocycle with the nitrogen to which they are attached;

ix) $-NR^2R^3$;

x) 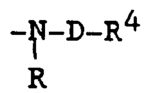

R is hydrogen or $C_{1-4}$ alkyl,

D is 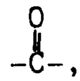, 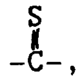, 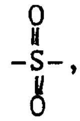, 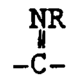, and

$R^4$ is NHR, $C_{1-3}$ alkyl, $C_{1-4}$ alkoxy, or $NR^2R^3$;

xi) $C_{3-7}$ cycloalkyl or $C_{6-10}$ aryl;

xii) 5 or 6 membered heterocycle, unsubstituted or substituted with -OH, NHR, or $C_{1-4}$ alkyl; or

xiii) aryl of 6-10 carbon atoms, either unsubstituted or substituted with one or more of

(a) halo,

(b) hydroxy,

(c) $C_{1-3}$ alkoxy,

(d) $C_{1-3}$ alkyl,

(e) $-NR_2$, wherein R is defined above,

(f) 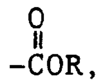,

(g) 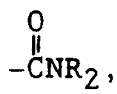,

(h) $-SO_2NR_2$,

(i) $-CH_2NR_2$,

(j) 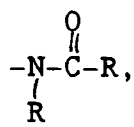, or

(k) 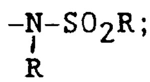;

xiv) $X[(CH_2)_mO]_nR$ where $X=CH_2$, NR, O, S, SO or $SO_2$; n=1-6, m may be 1-3 within each repeating unit n, and R is defined above;

b) aryl of 6-10 carbon atoms, either unsubstituted or substituted with one or more of

i) $C_{1-4}$ alkyl,

ii) $C_{1-3}$ alkoxy,

iii) hydroxy, or

iv) halo;

v) $-NR_2$,

vi) 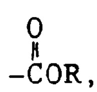,

vii) 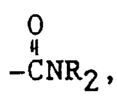,

viii) $-SO_2NR_2$,

ix) $-CH_2NR_2$,

x) -NRCOR, or

xi) $-NRSO_2R$;

xii) $X[(CH_2)_mO]_nR$ where $X=CH_2$, NR, O, S, SO or $SO_2$; n=1-6, m may be 1-3 within each repeating unit n, and R is defined above;

xiii) $CH_2X[(CH_2)_mO]_nR$;

c) 5 or 6 membered heterocycle;

3) $R^1-SO_2-$,

4) 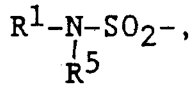 wherein

$R^5$ is H or $C_{1-5}$ alkyl or joined together with $R^1$ either directly to form 5-7 membered heterocycle, or through a heteroatom selected from N, O, and S, to form a 6-membered heterocycle with the nitrogen to which they are attached;

5) 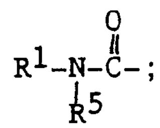

6) 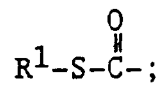

7) 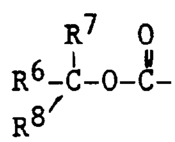

wherein $R^6$, $R^7$, and $R^8$

a) H,

b) $C_{1-6}$ alkyl unsubstituted or substituted with one or more of

i) halo,

ii) OH,

iii) aryl $SO_2$-,

iv) $-O-[(CH2)_mO]_n-R$,

c) Aryl unsubstituted or substituted with one or more of

i) $C_{1-4}$ alkyl,

ii) $C_{1-3}$ alkoxy,

iii) halo,

iv) nitro,

v) acetoxy,

vi) dimethylaminocarbonyl,

vii) phenyl,

viii) $C_{1-3}$ alkoxycarbonyl

d) fluorenyl,

e) $R^6$, $R^7$, and $R^8$ may be independently joined to form a monocyclic, bicyclic, or tricyclic ring system which is $C_{3-10}$ cycloalkyl and may be substituted with $C_{1-4}$ alkyl,

f) a 5-7 membered heterocycle;

G is

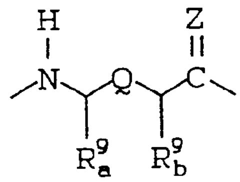

wherein Z is O, S, or NH and
$R^9_a$ or $R^9_b$ is independently

1) 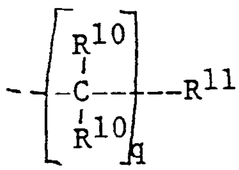, or

2) $C_{1-4}$ alkylene $-R^{11}$;
wherein q is 0-5 and $R^{10}$ is independently
a) hydrogen,
b) hydroxy, or
c) $C_{1-4}$alkyl;

provided that $R^9_a$ or $R^9_b$ is always substituted with $R^{12}$.

$R^{11}$ is

a) $C_6$-$C_{10}$ aryl, unsubstituted or substituted with $R^{12}$ and optionally with one or more of
i) halo,
ii) $C_{1-4}$alkyl,
iii) $C_{1-3}$ alkoxy;

b) monocyclic or bicyclic aromatic heterocyle containing from 1 to 3 heteroatoms chosen from N,O,S and which may be substituted with $R^{12}$ and optionally with one or more of
i) halo,
ii) $C_{1-4}$ alkyl,
iii) $C_{1-3}$ alkoxy;

c) $C_{1-6}$ alkyl or $C_{1-6}$ alkenyl unsubstituted or substituted with $R^{12}$ and optionally with one or more of
i) hydroxy,
ii) $C_{1-4}$ alkyl,
iii) $-NH_2$, -NHR, $-NR_2$,

iv) 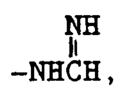,

v) 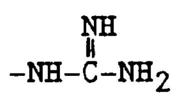,

vi) -COOH,

vii) 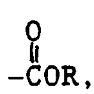,

viii) SR, S(O)R and $S(O_2)R$,
ix) $-SO_2NHR$,
x) $C_{1-4}$ alkyl sulfonyl amino or aryl sulfonyl amino,
xi) -CONHR,

xii) 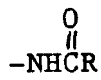,

xiii) -OR,

xiv) aryl $C_{1-3}$ alkoxy or,

xv) aryl;

d) $C_{3-7}$ cycloalkyl unsubstituted or substituted with $R^{12}$, and optionally with one or more of

i) hydroxy,

ii) $C_{1-4}$ alkyl,

iii) $-NH_2$, -NHR, $-NR_2$,

iv) 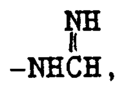,

v) 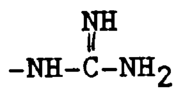,

vi) -COOH,

vii) 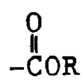,

viii) SR, S(O)R and $S(O_2)R$,

ix) $-SO_2NHR$,

x) $C_{1-4}$ alkyl sulfonyl amino or aryl sulfonyl amino,

xi) -CONHR,

xii) 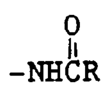; and

$R^{12}$ is

a) $X[((CH_2)_mO)_n]R^{13}$ where $X=CH_2$, NR, O, S, SO or $SO_2$; n=1-6, m may be 1-3 within each repeating unit n, and $R^{13}$ is selected from

i) H or $C_{1-4}$ alkyl,

ii) $C(=O)R^1$,

iii) $C(=O)X'[(CH_2)_mO]_nR$ wherein X' is NR or O,

iv) $P(=O)(OM)_2$ where M is a mono or divalent metal ion,

v) $C(=O)OR^1$,

vi) $R^1R^5NC(=O)$;

b) $CH_2X[((CH_2)_mO)_n]R^{13}$,

$R^{14}$ is -OH or $-NHR^{15}$, wherein $R^{15}$ is -H,

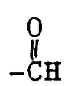,

$-C_{1-4}-$ alkyl, or -COOR; and

Q is

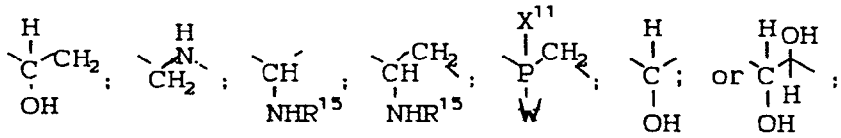

wherein $R^{15}$ is defined above;
$X^{11}$ is O, S or NH; and
W is
1) OH,
2) $NH_2$,
3) OR, or
4) NHR;
B is, independently, absent, or

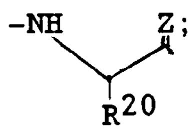

where $R^{20}$ is a hydrophobic group and is
a) $-CH(CH_3)_2$
b) $-CH(CH_3)(CH_2CH_3)$, or
c) -Phenyl;
J is
1) $YR^{16}$ wherein:
Y is 0 or NH, and
$R^{16}$ is
a) H;
b) $C_{1-6}$ alkyl, unsubstituted or substituted with one or more of
i) $-NR_2$,
ii) -OR,
iii) $-NHSO_2C_{1-4}$ alkyl,
iv) $-NHSO_2$ aryl, or $-NHSO_2$(dialkylaminoaryl),
v) $-CH_2OR$,
vi) $-C_{1-4}$ alkyl,

vii) 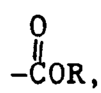

viii) 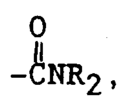

ix) 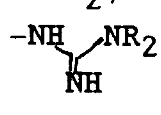 or 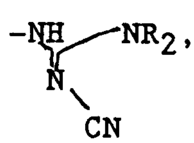

x) 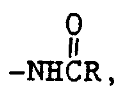

xi) 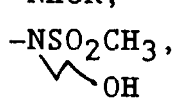

xii) 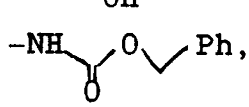

xiii) $-NR_3^{\oplus} A^{\ominus}$ wherein $A^{\ominus}$ is a counterion,
xiv) $-NR^{17}R^{18}$ wherein $R^{17}$ and $R^{18}$ are the same or different and are $C_{1-5}$ alkyl joined together directly to form a 5-7 membered heterocycle containing up to one additional heteroatom selected from -O-, -S-, or -NR-,
xv) aryl,
xvi) -CHO,
xvii) $-OP(O)(OR_x)_2$ wherein $R_x$ is H or aryl, or

xvii) $-O-\overset{O}{\overset{\|}{C}}-C_{1-4}alkyl$

substituted with one or more of amine or quaternary amine, or $-O-[(CH_2)_mO]_n-R$, or $-OP(O)(OR_x)_2$;
c) $-[(CH_2)_mO]_nCH_3$ or $-[(CH_2)_mO]_n H$;
2) $N(R^{16})_2$;
3) $-NR^{17}R^{18}$ wherein $R^{17}$ and $R^{18}$ are defined above; or

4) 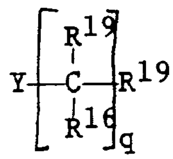

wherein: Y, $R^{16}$ and q are defined above, and
$R^{19}$ is
a) hydrogen;
b) aryl unsubstituted or substituted with one or more of
i) halo,
ii) -OR, wherein R is H or $C_{1-4}$ alkyl,

iii) $-\overset{O}{\overset{\|}{C}}OR$,

iv) $-\overset{O}{\overset{\|}{C}}NR_2$,

v) $-CH_2NR_2$,
vi) $-SO_2NR_2$,
vii) $NR_2$,

viii) $-NH\overset{O}{\overset{\|}{C}}R$,

xi) $C_{1-4}$ alkyl,
x) phenyl
xi) $-CF_3$,

xii) $-\overset{R}{\overset{|}{N}}-SO_2R$,

xiii) $-C_{1-4}$ alkyl $-NR_2$,
xiv) $-OP(O)(OR_x)_2$ wherein $R_x$ is H or aryl, or

xv) $-O-\overset{O}{\overset{\|}{C}}-C_{1-4}alkyl$

substituted with one or more of amine or quaternary amine, or $-OP(O)(OR_x)2$;

c) A 5-7 membered heterocyclic ring or, 7-10 membered bicyclic heterocyclic ring which is saturated or unsaturated, the ring being unsubstituted or substituted with one or more of

i) halo,

ii) -OR, wherein R is H, $C_{1-4}$alkyl, or $C_{1-4}$alkenyl,

iii) $-\overset{O}{\overset{\|}{C}}OR$,

iv) $-\overset{O}{\overset{\|}{C}}NR_2$,

v) $-CH_2NR_2$,

vi) $-SO_2NR_2$,

vii) $-NR_2$,

viii) $-NH\overset{O}{\overset{\|}{C}}R$,

xi) $C_{1-4}$ alkyl,

x) phenyl

xi) $-CF_3$,

xii) $-\overset{R}{\overset{|}{N}}-SO_2R$,

xiii) phenyl $C_{1-4}$ alkyl,

xiv) $-OP(O)(OR_x)_2$ wherein $R_x$ is H or aryl,

xv) $-O-\overset{O}{\overset{\|}{C}}-C_{1-4}alkyl$

substituted with one or more of amine or quaternary amine, or $-OP(O)(OR_x)_2$, or $-O[(CH_2)_mO]_n-R$, or

xvi) $-O-\overset{O}{\overset{\|}{C}}-O-[(CH_2)_mO]_n-R$;

d) A 5 to 7 membered carbocyclic or 7-10 membered bicyclic carbocyclic ring which is either saturated or unsaturated, the carbocyclic ring being unsubstituted or substituted with one or more of

i) halo,

ii) -OR, wherein R is H or $C_{1-4}$ alkyl,

iii) 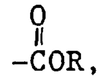

iv) 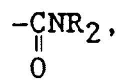

v) $-CH_2NR_2$,
vi) $-SO_2NR_2$,
vii) $-NR_2$,

viii) 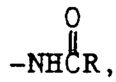

xi) $C_{1-4}$ alkyl,
x) phenyl,
xi) $-CF_3$,

xii) 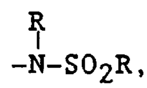

xiii) $-OP(O)(OR_x)_2$ wherein $R_x$ is H or aryl,

xiv) 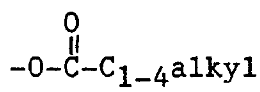

substituted with one or more of amine or quaternary amine, $-OP(O)(OR_x)_2$, or $-O-[(CH_2)_mO]_n-R$, or

xv) 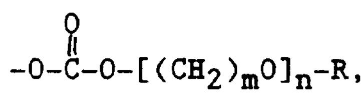

or pharmaceutically acceptable salts thereof.

2. A compound of Claim 1, wherein:
A is:

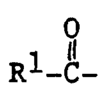 or 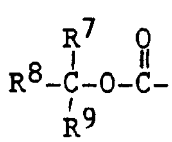, and

G is:

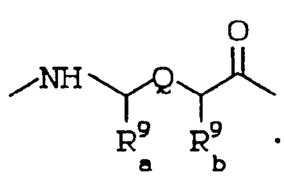

3. A compound of Claim 2, wherein:

B is absent or present once, and
Q is

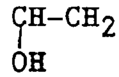

.

4. A compound of Claim 3,
wherein:
B is absent, J is $NHR^{19}$ and $R^{19}$ is a substituted 7- to 10-membered bicyclic carbocycle or heterocycle which may be saturated or unsaturated.

5. A compound of Claim 4,
wherein:
J is indanyl, benzo[c] thiopyranyl-2,2-dioxide, or cyclopentyl, any of which are unsubstituted or substituted.

6. A compound, which is
N-(2(R)-hydroxy-1(S)-indanyl)-5(S)-(1,1-dimethylethyloxycarbonylamino)-4(S)-hydroxy-2(R)-(4-(1,4,7-trioxaoctyl)phenyl)methyl-6-phenylhexanamide,
N-(2(R)-hydroxy-1(S)-indanyl)-5(S)-(1,1-dimethylethoxycarbonyl)amino-4(S)-hydroxy-6-phenyl-2(R)-4-(2-hydroxyethoxy)phenylmethylhexanamide,
N-(2(R)-hydroxy-1(S)-indanyl)-5(S)-(1,1-dimethylethoxycarbonyl)amino-4(S)-hydroxy-6-phenyl-2(R)-4-(2-hydroxyethyl)phenylmethylhexanamide,
N-(2(R)-hydroxy-1(S)-indanyl)-5(S)-(1,1-dimethylethoxycarbonyl)amino-4(S)-hydroxy-6-phenyl-2(R)-4-(3-hydroxypropyl)phenylmethylhexanamide,
N-(2(R)-hydroxy-1(S)-indanyl)-5(S)-(1,1-dimethylethoxycarbonyl)amino-4(S)-hydroxy-6-phenyl-2(R)-4-(3-(2-methoxyethoxy)methoxypropyl)phenylmethylhexanamide,
[5(S)-(1,1-dimethylethoxycarbonylamino)-4(S)-hydroxy-6-phenyl-2(R)-4-(2-hydroxyethoxy)phenylmethylhexanoyl]-valine-N-3,6,9,12-tetraoxatridecylamide,
[5(S)-(1,1-dimethylethoxycarbonylamino)-4(S)-hydroxy-6-phenyl-2(R)-4-(2-hydroxyethoxy)phenylmethyl hexanoyl]-valine-N-3,6,9,12,15-pentaoxapentadecylamide,
N-(2(R)-hydroxy-1(S)-indanyl)-5(S)-(1,1-dimethylethoxycarbonylamino)-4(S)-hydroxy-6-phenyl-2(R)-[4-(1,4,7,10-tetraoxaundecyl)phenyl]methyl hexanamide,
N-(2(R)-(hydroxy-1(S)-indanyl)-5(S)-(1,1-dimethylethoxycarbonylamino)-4(S)-hydroxy-6-phenyl-2(R)-[4-(methoxyethoxyethoxy)phenyl]methylhexanamide,
N-(2(R)-hydroxy-1(S)-indanyl)-5(S)-(1,1-dimethylethoxycarbonylamino)-4(S)-hydroxy-6-phenyl-2(R)-[4-(methoxyethoxy) phenyl] methyl hexanamide,
N-(2(R)-hydroxy-1(S)-indanyl)-5(S)-(1,1-dimethylethoxycarbonylamino)-4(S)-hydroxy-6-phenyl-2(R)-[4-(1,4,7,10,13-pentoxatetradecyl) phenyl] methyl hexanamide,
N-(4-(3,4-Dihydro-(1H)-benzo[c]thiopyranyl-2,2-dioxide))-5(S)-(1,1-dimethylethoxycarbonylamino)-4(S)-hydroxy-6-phenyl-2(R)-4(3-hydroxypropyl)phenylmethylhexanamide,
N-(2(R)-hydroxy-1(S)-indanyl)-5(S)-(1,1-dimethylethyloxycarbonylamino)-4(S)-hydroxy-2(R)-(5-thia-7-hydroxy-2(E)-hepten-1-yl)-6-phenylhexanamide,
N-(2(R)-hydroxy-1(S)-indanyl)-5(S)-(1,1-dimethylethyloxycarbonylamino)-4(S)-hydroxy-2(R)-(5-thia-8-hydroxy-2(E)-octen-1-yl)-6-phenylhexanamide,
N-(2(R)-hydroxy-1(S)-indanyl)-5(S)-(1,1-dimethylethyloxycarbonylamino)-4(S)-hydroxy-2(R)-4-(4-hydroxy-2-thiabut-1-yl)phenylmethyl-6-phenylhexanamide,
N-(2(R)-hydroxy-1(S)-indanyl)-5(S)-(1,1-dimethylethyloxycarbonylamino)-4(S)-hydroxy-2(R)-(5,8,11,14,17-pentaoxaoctadec-2(E)-en-1-yl)-6-phenylhexanamide,
N-(2(R)-hydroxy-1(S)-indanyl)-5(S)-(1,1-dimethylethyloxycarbonylamino)-4(S)-hydroxy-2(R)-4-(2-hydroxyethylsulfonylmethyl)phenylmethyl-6-phenylhexanamide,
N-(2(R)-hydroxy-1(S)-indanyl)-5(S)-(1,1-dimethylethyloxycarbonylamino)-4(S)-hydroxy-2(R)-4-(2,5,8,11,14-pentaoxapentadec-1-yl)phenylmethyl-6-phenylhexanamide,
N-(2(R)-hydroxy-1(S)-indanyl)-5(S)-(1,1-dimethylethyloxycarbonylamino)-4(S)-hydroxy-2(R)-(4-(2-(hydroxy) ethoxy)phenyl)methyl-6-cyclohexyl-hexanamide,
N-(4-fluoro-2(R)-hydroxy-1(S)-indanyl)-5(S)-(1,1-dimethylethyloxycarbonylamino)-4(S)-hydroxy-2(R)-(4-(2-hydroxyethyloxy)phenylmethyl-6-phenylhexanamide,

N-(1-hydroxy-3-methyl-2-cyclopentyl)-5(S)-(1,1-dimethylethoxycarbonyl)amino-4(S)-hydroxy-6-phenyl-2(R)-[4-(2-hydroxyethoxy)phenyl]methylhexanamide,
N-(1-hydroxy-3-methyl-2-cyclopentyl)-5(S)-(1,1,-dimethylethoxycarbonylamino)-4(S)-hydroxy-6-phenyl-2(R)-[4-(2-methoxyethoxy)phenyl] methyl hexanamide,
N-(2(R)-hydroxy-1(S)-indanyl)-5(S)-(1,1-dimethylethoxycarbonylamino)-4(S)-hydroxy-6-phenyl-2(R)-[4-(1,4,6,9,12,15,18-heptoxa-5-oxo-nonadecyl)phenyl]methylhexanamide,
N-(2(R)-hydroxy-1(S)-indanyl)-5(S)-(1,1-dimethyl ethoxycarbonylamino)-4(S)-hydroxy-6-phenyl-2(R)-[4-(6-aza-1,4,9,12,15,18-hexoxa-5-oxo-nonadecyl)phenyl]methylhexanamide,
N-(2(R)-hydroxy-1(S)-indanyl)-5(S)-(1,1-dimethylethoxycarbonylamino)-4(S)-hydroxy-6-phenyl-2(R)-[4-(2-phosphoryloxyethoxy)phenyl]methyl hexanamide, or
N-(2(R)-hydroxy-1(S)-indanyl)-5(S)-(1,1-dimethylethoxycarbonylamino)-4(S)-hydroxy-6-phenyl-2(R)-[4-(2-phosphoryloxypropyl) phenyl]methyl hexanamide,
N-(2(R)-hydroxy-1(S)-indanyl)-5(S)-(3-oxabutyloxycarbonylamino)-4(S)-hydroxy-2(R)-(4-(2-hydroxy)ethoxyphenyl)methyl-6-phenylhexanamide,
N-(2(R)-hydroxy-1(S)-indanyl)-5(S)-(3,6-dioxaheptyloxycarbonylamino)-4(S)-hydroxy-2(R)-((4-(2-hydroxy)ethoxy)phenyl)methyl-6-phenylhexanamide,
N-(2(R)-hydroxy-1(S)-indanyl)-5(S)-(3,6,9,12-tetraoxa-tridecyloxycarbonylamino)-4(S)-hydroxy-6-phenyl-2(R)-[4-(1,4,7-trioxaoctyl)phenyl]methylhexanamide,
N-(2(R)-hydroxy-1(S)-indanyl)-5(S)-(3,6-dioxaheptyloxycarbonylamino)-4(S)-hydroxy-6-phenyl-2(R)-[4-(1,4-dioxapentyl)phenyl]methylhexanamide,
N-(2(R)-hydroxy-1(S)-indanyl)-5(S)-(3,6-dioxaheptyloxycarbonylamino)-4(S)-hydroxy-6-phenyl-2(R)-[4-(1,4, 7-trioxaoctyl)phenyl]methylhexanamide,
N-(2(R)-hydroxy-1(S)-indanyl)-5(S)-(3,6,9-trioxadecyloxycarbonylamino)-4(S)-hydroxy-6-phenyl-2(R)-[4-(1,4,7-trioxaoctyl)phenyl]methylhexanamide,
N-(2(R)-hydroxy-1(S)-indanyl)-5(S)-(3,6,9,12-tetraoxa-tridecyloxycarbonylamino)-4(S)-hydroxy-6-phenyl-2(R)-[4-(1,4-dioxapentyl)phenyl]methylhexanamide,
N-(2(R)-hydroxy-1(S)-indanyl)-5(S)-(2-(2-methoxyethoxy)ethoxycarbonylamino)-4(S)-hydroxy-2(R)-(4-(3(2-methoxyethoxy)methoxy)propyl)phenylmethyl-6-phenylhexanamide,
N-(2(R)-hydroxy-1(S)-indanyl)-5(S)-(3,6,9-trioxadecyloxycarbonylamino)-4(S)-hydroxy-6-phenyl-2(R)-[4-(3,6,9,12-tetraoxatridecyloxy)phenyl]methylhexanamide,
N-(2(R)-hydroxy-1(S)-indanyl)-5(S)-(3,6,9,12-tetraoxatridecyloxycarbonylamino)-4(S)-hydroxy-6-phenyl-2(R)-[4-(3,6,9,12-tetraoxatridecyloxy)phenyl]methylhexanamide,
N-(2-(R)-hydroxy-1(S)-indanyl)-5(S)-(1,1-dimethylethoxycarbonylamino)-4(S)-hydroxy-2(R)-(4-(2-phosphoryloxyethoxy)phenyl)methyl-6-cyclohexylhexanamide,
or pharmaceutically acceptable salts thereof.

7. The compound according to Claim 6, which is N-(2(R)-hydroxy-1(S)-indanyl)-5(S)-(1,1-dimethylethyloxycarbonylamino)-4(S)-hydroxy-2(R)-(4-(2-(hydroxy)ethoxy)phenyl)methyl-6-cyclohexylhexanamide, or pharmaceutically acceptable salts thereof.

8. The compound according to Claim 6, which is N-(2(R)-hydroxy-1(S)-indanyl)-5(S)-(1,1-dimethylethyloxycarbonylamino)-4(S)-hydroxy-2(R)-(4-(1,4,7-trioxaoctyl)phenyl)methyl-6-phenylhexanamide, or pharmaceutically acceptable salts thereof.

9. The compound according to Claim 6, which is N-(2(R)-hydroxy-1(S)-indanyl)-5(S)-(1,1-dimethylethyloxycarbonylamino)-4(S)-hydroxy-2(R)-(4-(2-(hydroxy)-ethoxy)phenyl)methyl-6-phenylhexanamide, or pharmaceutically acceptable salts thereof.

10. The compound N-(2(R)-hydroxy-1(S)-indanyl)-5(S)-(3,6-dioxaheptyloxycarbonylamino)-4(S)-hydroxy-2(R)-((4-(2-hydroxy)ethoxy)phenyl)methyl-6-phenylhexanamide, or pharmaceutically acceptable salt thereof.

11. The compound N-(2(R)-hydroxy-1(S)-indanyl)-5(S)-(2-(2-methoxyethoxy)ethoxycarbonylamino)-4(S)-hydroxy-2(R)-(4-(3-(2-methoxyethoxy)methoxy)propyl)phenylmethyl-6-phenylhexanamide, or pharmaceutically acceptable salt thereof.

12. The compound N-(2-(R)-hydroxy-1(S)-indanyl)-5(S)-(1,1-dimethylethoxycarbonylamino)-4(S)-hydroxy-2(R)-(4-(2-phosphoryloxyethoxy)phenyl)methyl-6-cyclohexylhexanamide,

or pharmaceutically acceptable salt thereof.

13. A pharmaceutical composition useful for inhibiting HIV protease, comprising an effective amount of a compound as in any one of Claims 1-12, and a pharmaceutically acceptable carrier.

14. A pharmaceutical composition useful for preventing or treating infection by HIV or for treating AIDS or ARC, comprising an effective amount of a compound as in any one of Claims 1-12, and a pharmaceutically acceptable carrier.

15. The use of a compound as claimed in any one of Claims 1 to 12 for the manufacture of a medicament for inhibiting HIV protease.

16. The use of a compound as claimed in any one of Claims 1 to 12 for the manufacture of a medicament for preventing infection by HIV, or of treating infection by HIV or of treating AIDS or ARC.